# EUROPEAN PATENT APPLICATION

(11) **EP 3 543 244 A1**
(43) Date of publication of application: **25.09.2019**
(21) Application number: 18163477.5
(22) Date of filing: 22.03.2018
(51) Int. Cl.: C07D 493/04, C08K 5/06

(54) **DIACETAL DERIVATIVES AND THEIR USE AS CLARIFIER**

(71) Applicant: Clariant International Ltd, 4132 Muttenz (CH)
(72) Inventor: HAUCK. Stefan, 64665 Alsbach-Haehnlein (DE); BUDDE, Felix, 60326 Frankfurt am Main (DE); MULLER, Thierry, 9026 Ettelbruck (LU)
(74) Representative: Paczkowski, Marcus

(57) **Abstract**

The invention relates to a nucleating agent of formula (I) 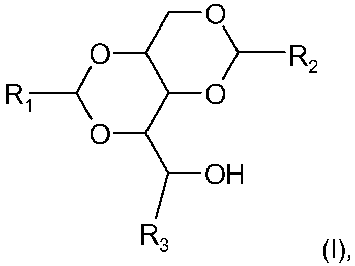 wherein residues R₁ and R₂ are independently selected from substituted phenyl groups, wherein the substituents of said substituted phenyl groups are independently from each other selected from the group consisting of (C₁-C₄)-alkyl-; and wherein R₃ is a -C(O)OR4, -C(O)NR₄R₅, or -C(O)-NR₇-NR₅R₆ residue, wherein R₄, R₅, R₆, and R₇ are independently from each other hydrogen or (C₁-C₄)-alkyl-.

## Description

The invention relates to nucleating agents of formula (I) useful to improved haze and clarity

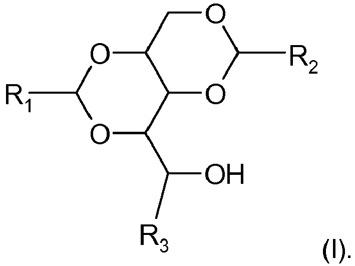

Nucleating agents reduce the haze in articles made from crystalline polymers. In crystalline thermoplastics, nucleating agents are used to influence the spherulite size and to achieve earlier solidification of the polymer melt. A reduction in spherulite size usually improves the transparency of the materials and their mechanical properties. An earlier solidification of the polymer melt leads for example to shorter cycle times in injection molding processes. Further the stiffness and temperature resistance of the polymeric articles can be improved by the use of nucleating agents.

Common nucleating agents are acetals of sorbitol and xylitol, which have been employed as clarifying agents (US 4,016,118; US 4,314,039; US 4,371,645; US 4,954,291; US 5,049,605). In US 4,016,118 dibenzylidene sorbitol (DBS) is disclosed. Substitution of various groups upon the benzyl moieties of DBS are suggested to improve their ability to reduce haze in and improve transparency of polyolefin articles. US 4,314,039 for example proposes the use of 1,3:2,4 di(alkylbenzylidene) sorbitols, US 4,371,645 describes dibenzylidene sorbitols having at least one chlorine or bromine as substituent in the benzyl groups and US 4,954,291 discloses mixtures of different sorbitol acetals or xylitol acetals. Representative DBS derived nucleating agents of the state of the art are for example bis(3,4-dialkyl-benzylidene) sorbitol acetals (US 5,049,605).

In US 2005/0239926; US 2005/0239928 and 2007/0299256 1,3:2,4-dibenzylidene sorbitols or xylitols are described as nucleating agents, having an additional residue selected from alkyl groups, alkenyl groups, alkoxy groups, hydroxyalkyl groups and alkyl-halide groups in position 1.

A further class of nucleating agents described in US 5,283,275 are dibenzylidene xylonates. The use of 3,5:4,6 protected derivatives of gulonic acid for the preparation of 2-keto gulonic acid, its ester or of ascorbic acid is described in EP 0 000 243 B1. Dibenzylidenated polyhydric alcohol derivatives, including derivatives of gulonic acid, are further used for the preparation of uniform oil-in-polyol emulsions according to JP H0267285A.

As chemical arts often are unpredictable, modification of substituents can have a significant impact upon the performance and utility of the compound. In the past different substituents and substitution patterns of the benzylidene rings of DBS derivatives are tested as nucleating agents. However, further improvements are still desired to develop nucleating agents that are able to reduce haze and to increase clarity when used as plastic additives in polymer compositions. Desirably the nucleating agents can be easily modified with functional groups allowing a customization of the properties of the nucleating agent in a way that adjustment of their performance to special polymer types can be achieved. Beside reduced haze and increased clarity for example good blending characteristics or stabilization effects of the nucleating agents may be of interest.

The problem is solved by dibenylidene gulonic acid derivatives, in particular by 3,5:4,6-dibenzylidene *D*- or *L*-gulonic acid derivatives. Suitable sugar acid derivatives are those of formula (I).

This invention provides a variety of easily available dibenzylidene sugar acid derivatives, such as esters, carbonic acids, amides or hydrazides, which allow a customization of the additive properties in a way that adjustment of their performance to special polymer types can be achieved. Further, reduced haze and in parallel higher clarity enhance the optical properties of a plastic article containing said additives in a way that products seen through e.g. a packing film are perceived as sharp and detailed.

Accordingly, a first embodiment of the present invention is a nucleating agent of formula (I): 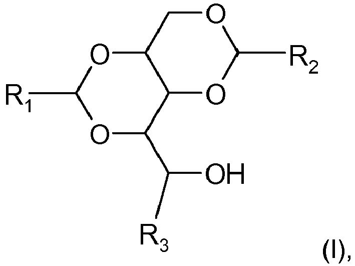 wherein
residues R₁ and R₂ are independently selected from substituted phenyl groups, wherein the substituents of said substituted phenyl groups are independently selected from the group consisting of (C₁-C₄)-alkyl-;
and wherein
R₃ is a residue of formula (II), (III) or (IV) 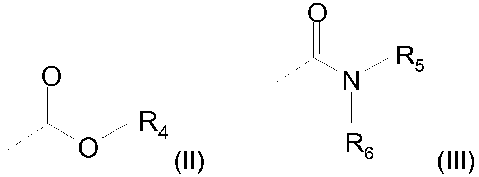 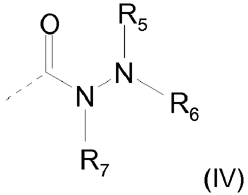 wherein
R₄, R₅, R₆, and R₇ are independently from each other hydrogen or (C₁-C₄)-alkyl-.

The nucleating agents of the invention may be used in polymer layers, films, sheets or foils which are solid at room temperature (at 20°C), preferably these polymer articles are solid up to at least 70°C or most preferred up to at least 100°C. The polymer layers, films, sheets or foils can be part of a multilayer composition e. g. in packaging materials and container materials for food, pharmaceutical compositions, cosmetics etc., in particular, for transparent packages or containers.

Alkyl radicals in the compounds of formula (I) have between preferably between 1 and 4 carbon atoms and may be unbranched or branched. Examples of alkyl radicals are methyl, ethyl, n-propyl, isopropyl, n-butyl, isobutyl, sec-butyl and tert-butyl. Particularly preferred are methyl and ethyl.

Residues R₃ of formula (II), (III) or (IV) are bound to the compounds of formula (I) or (Ia) via the bond indicated as discontinued line ("-----").

The nucleating agents of the present invention can be used for example as clarifier in semicrystalline resins, in particular semicrystalline thermoplastic resins. Preferred polymers are selected from the group consisting of polyolefin homopolymers, polyolefin copolymers and polyamides or mixtures thereof. In particular preferred is the use of the nucleating agents in polymers selected from the group consisting of olefinic homopolymers derived from aliphatic olefins with 2 to 6 carbon atoms or olefinic copolymers derived from at least one aliphatic olefin with 2 to 6 carbon atoms and one or more ethylenically unsaturated comonomers, such as polyethylene, polytetrafluoroethylene, polypropylene, polybutylene, polyisobutylene, polyisoprene, polybutadiene, polyethylene terephthalate, polypropylene terephthalate, polybutylene terephthalate, copolymers of ethylene and an ethylenically unsaturated comonomer selected from the group of propylene, 1-butylene, isobutylene, 1-pentylene, 1-hexylene, isoprene, (C₃-C₇)-cycloalkene, norbornene and styrene, copolymers of propylene and an ethylenically unsaturated comonomer selected from the group of ethylene, 1-butylene, isobutylene, 1-pentylene, 1-hexylene, isoprene, (C₃-C₇)-cycloalkene, norbornene and styrene; or polyamides, such as polyamide-6,6, and polyamide-6; or mixtures thereof. The nucleating agents of the present invention are particularly useful in polypropylene (PP), including isotactic or syndiotactic polypropylene, polyethylene, including LDPE, LLDPE and HDPE, polyethylene terephthalate (PET), syndiotactic polystyrene, nylon 6, and nylon 6,6.

The mentioned polymers may be blended with up to 15 wt.-% of additional polymers such as polyacrylates, vinylacetate copolymers, polyvinylacohols etc.

The described nucleating agents are useful in thermoplastic polymers with a melting point between 70°C and 250°C, preferably between 100°C and 200°C. The melting point of the polymers can be determined by differential scanning calorimetry (DSC) performed on a Mettler Toledo DSC 3+ under nitrogen atmosphere in an alumina 40 µl crucible with a standard heating and cooling rate of 10 K/min. Prior to recording of the DSC thermogram, 10 mg of the polymer is held 20°C above the melting temperature for 5 min. Then, the measurement of the melting point is carried out in a two-cycle heating-cooling experiment. The melting point of the polymer is the peak temperature of the 2^{nd} heating in the corresponding thermograms.

Preferred nucleating agents of formula (I) are selected from 3,5:4,6-dibenzylidene gulonic acid derivatives, wherein the residues R₁ and R₂ of the acetal groups are selected from monosubstituted, disubstituted, trisubstituted, tetrasubstituted or pentasubstituted phenyl groups, wherein mono-, di- and trisubstituted phenyl groups are particularly preferred. The substituents of said substituted phenyl groups are independently from each other selected from the group consisting of (C₁-C₄)-alkyl-.

The alkyl radical of the mono-substituted phenyl groups is preferable present in position 3 or 4 (3-(C₁-C₄)-alkyl phenyl or 4-(C₁-C₄)-alkyl phenyl). The alkyl radicals of the di-substituted phenyl groups are preferably present in position 2, 3, 4 or 5, particularly 2,4-di-(C₁-C₄)-alkyl phenyl, 2,5-di-(C₁-C₄)-alkyl-phenyl, 3,4-di-(C₁-C₄)-alkyl phenyl or 3,5-di-(C₁-C₄)-alkyl phenyl groups are of interest. Preferred tri-substituted phenyl groups are 3,4,5-tri-(C₁-C₄)-alkyl phenyl and 2,3,4-tri-(C₁-C₄)-alkyl phenyl.

Examples of such groups are 4-methylphenyl, 4-ethylphenyl, 4-propylphenyl, 4-isobutylphenyl, 3-methylphenyl, 3-ethylphenyl, 3-propylphenyl, 3-isobutylphenyl, 3,4-dimethylphenyl, 3,5-dimethylphenyl, 2,4-dimethylphenyl, 2,5-dimethylphenyl, 3,4-diethylphenyl, 3,5-diethylphenyl, 2,4-diethylphenyl, 2,5-diethylphenyl, 3,4-diproylphenyl, 3,5-dipropylphenyl, 2,4-dipropylphenyl, 2,5-dipropylphenyl, 3,4-diisopropylphenyl, 3,5-diisopropylphenyl, 2,4-diisopropylphenyl, 2,5-diisopropylphenyl, 3-ethyl-4-methyl-phenyl, 3-ethyl-5-methyl-phenyl, 2-ethyl-4-methyl-phenyl, 2-ethyl-5-methyl-phenyl, 3-methyl-4-ethyl-phenyl, 3-methyl-5-ethylphenyl, 2-methyl-4-ethyl-phenyl, 2-methyl-5-ethyl-phenyl, 3-propyl-4-methyl-phenyl, 3-propyl-5-methyl-phenyl, 2-propyl-4-methyl-phenyl, 2-propyl-5-methyl-phenyl, 3-methyl-4-propyl-phenyl, 3-methyl-5-propyl-phenyl, 2-methyl-4-propyl-phenyl, 2-methyl-5-porpyl-phenyl, 3-ethyl-4-propyl-phenyl, 3-ethyl-5-propyl-phenyl, 2-ethyl-4-propyl-phenyl, 2-ethyl-5-propyl-phenyl, 3-propyl-4-ethyl-phenyl, 3-propyl-5-ethylphenyl, 2-propyl-4-ethyl-phenyl, and 2-propyl-5-ethyl-phenyl. Preferred trisubstituted phenyl groups are 3,4,5-trimethylphenyl and 2,3,4-trimethylphenyl.

Most preferred residues R₁ and R₂ are 4-methylphenyl, 4-ethylphenyl, 4-propylphenyl, 4-butylphenyl, including 4-isobutylphenyl, 3-methylphenyl, 3,4-dimethylphenyl, 3,5-dimethylphenyl, 2,4-dimethylphenyl, 2,5-dimethylphenyl. R₁ and R₂ may be different, but nucleating agents with R₁ = R₂ are preferred.

Preferred nucleating agents of the present invention derive from *L*-gulonic acid. Suitable *L*-gulonic acid derivatives are 3,5:4,6-dibenzylidene-*L*-gulonic acid derivatives of formula (Ia) 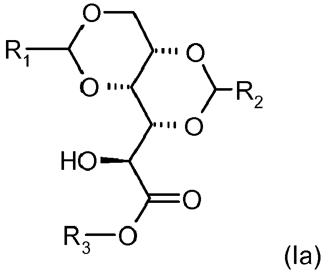 wherein the residues R₁, R₂ and R₃ have the same meaning as described above.

R₃ in compounds of formula (I) and (Ia) is a group selected from -C(O)OR₄ (formula (II)), -C(O)NR₅R₆ (formula (III)) or -C(O)-NR₇-NR₅R₆ (formula (IV)). The residues of R₄, R₅, R₆ and R₇ in these groups are independently from one another hydrogen or (C₁-C₄)-alkyl-. Preferred residues R₃ are selected from the residue of formula (II) with R₄ equal to hydrogen, methyl or ethyl, most preferred R₄ is hydrogen; or from the residue of formula (III) with R₅ equal to hydrogen and R₆ equal to hydrogen, methyl, ethyl, propyl or butyl, most preferred R₆ is hydrogen; R₅ and R₇ are hydrogen; and R₆ is hydrogen methyl, ethyl, propyl or butyl, most preferred R₆ is hydrogen, methyl or ethyl.

Particularly preferred are nucleating agent of formula (I) or (Ia) having a residue R₃ of formula (II), 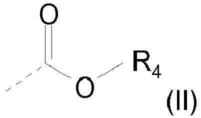 wherein
R₄ = H; the residue R₁ = R₂ and the residues R₁ and R₂ are selected from the group consisting of 4-methylphenyl, 3,4-dimethylphenyl, 3,5-dimethylphenyl, and 2,4-dimethylphenyl; or
a nucleating agent of formula (I) or (Ia) having a residue R₃ of formula (III), 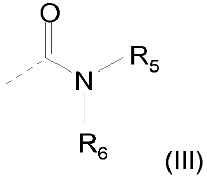 wherein
R₅ = H and R₆ = H, methyl or ethyl; the residue R₁ = R₂ and the residues R₁ and R₂ are selected from the group consisting of 4-methylphenyl, 4-ethylphenyl, 4-propylphenyl, 4-butylphenyl, 3,4-dimethylphenyl, 3,5-dimethylphenyl, and 2,4-dimethylphenyl; or
a nucleating agent of formula (I) or (Ia) having a residue R₃ formula (IV), 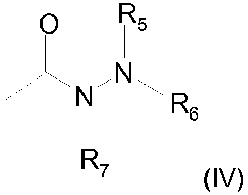 wherein
R₅ = H, R₇ = H and R₆ = H, methyl or ethyl; the residue R₁ = R₂ and the residues R₁ and R₂ are selected from the group consisting of 4-methylphenyl, 4-ethylphenyl, 4-propylphenyl, 4-butylphenyl, 3,4-dimethylphenyl, 3,5-dimethylphenyl, and 2,4-dimethylphenyl.

Examples of nucleating agent b) are 3,5:4,6-Di-(4-Methylbenzylidene)-L-gulonic acid methyl ester, 3,5:4,6-Bis-(3,4-Dimethylbenzylidene)-L-gulonic acid methyl ester, 3,5:4,6-Bis-(3,5-Dimethylbenzylidene)-L-gulonic acid methyl ester, 3,5:4,6-Bis-(2,5-Dimethylbenzylidene)-L-gulonic acid methyl ester, 3,5:4,6-Di-(4-Propylbenzylidene)-L-gulonic acid methyl ester, 3,5:4,6-Di-(4-Isobutylbenzylidene)-L-gulonic acid methyl ester, 3,5:4,6-Di-(4-Propylbenzylidene)-L-gulonic acid ethyl ester, 3,5:4,6-Di-(4-Methylbenzylidene)-L-gulonic acid, 3,5:4,6-Bis-(3,4-Dimethylbenzylidene)-L-gulonic acid, 3,5:4,6-Bis-(3,5-Dimethylbenzylidene)-L-gulonic acid, 3,5:4,6-Bis-(2,4-Dimethylbenzylidene)-L-gulonic acid, 3,5:4,6-Bis-(2,5-Dimethylbenzylidene)-L-gulonic acid, 3,5:4,6-Di-(4-Propylbenzylidene)-L-gulonic acid, 3,5:4,6-Di-(4-Isobutylbenzylidene)-L-gulonic acid, 3,5:4,6-Di-(4-Methylbenzylidene)-L-gulonic amide, 3,5:4,6-Bis-(3,4-Dimethylbenzylidene)-L-gulonic amide, 3,5:4,6-Bis-(3,5-Dimethylbenzylidene)-L-gulonic amide, 3,5:4,6-Bis-(2,4-Dimethylbenzylidene)-L-gulonic amide, 3,5:4,6-Bis-(2,5-Dimethylbenzylidene)-L-gulonic amide, 3,5:4,6-Di-(4-Propylbenzylidene)-L-gulonic amide, 3,5:4,6-Di-(4-Isobutylbenzylidene)-L-gulonic amide, 3,5:4,6-Bis-(3,4-Dimethylbenzylidene)-L-gulonic methyl amide, 3,5:4,6-Bis-(3,4-Dimethylbenzylidene)-L-gulonic ethyl amide, 3,5:4,6-Di-(4-Propylbenzylidene)-L-gulonic methyl amide, and 3,5:4,6-Di-(4-Propylbenzylidene)-L-gulonic ethyl amide.

The suitable amount of nucleating agent useful for the adjustment of the haze and for a maximum of clarity of the polymeric article can be easily determined and depends on the polymer and on the chosen nucleating agent. Usually the nucleating agent of formula (I) may be utilized in low concentrations, e. g. between 0.01 wt.-% and 10 wt.-%, preferably between 0.05 wt.- % and 2 wt.-%, most preferred between 0.1 wt.-% and 1 wt.-%, based on the amount of polymer.

The polymer composition to be treated with nucleating agents of the present invention may comprise further additives, such as fillers, antioxidants, stabilizers, plasticizers, lubricants, coloring agents, flame retardants, neutralizers, antiblock agents, antistatic materials, wetting agents, etc. Representative antioxidants for example include phenolic compounds, such as 2,6-di-t-butyl-p-cresol (BHT), tetrakis [methylene-3-(3,5-di-t-butyl-4-hydroxyphenyl) propionate] methane and n-octadecyl-3-(4'-hydroxy-3 ,5'-di-t-butylphenyl) propionate or phosphite compounds, such as bis(2,4-di-t-butylphenyl) pentaerythritol diphosphate and tris(2,4-di-t-butylphenyl) phosphite, representative ultraviolet light stabilizers include, but are not limited to, various substituted resorcinols, salicylates, benzotriazole, benzophenones, triazines and combinations thereof. Suitable lubricants include, but are not limited to, fatty acids, fatty acid esters or amides, fatty alcohols, such as stearic acid, stearyl alcohol, and stearamides. Antistatic agent includes, for example, glycerin esters of fatty acids having 8 to 22 carbon atoms, sorbitan esters, and polyethylene glycol esters. Exemplary flame-retardants include, but are not limited to, organic halogenated compounds, including decabromodiphenyl ether, halogen-free organic flame-retardants such as aluminum salts of diethylphosphinic acid, melamine cyanurate, melamin polyphosphate and inorganic compounds like ammonium polyphosphate, ATH and the like, and combinations thereof. Suitable coloring agents including dyes and pigments include, but are not limited to, cadmium sulfide, cadmium selenide, titanium dioxide, phthalocyanines, ultramarine blue, nigrosine, carbon black and combinations thereof. Representative oxidative and thermal stabilizers include, but are not limited to, metal halides, such as sodium halides, potassium halides, lithium halides, cuprous halides, as well as corresponding chlorides, bromides, and iodides, respectively, and combinations thereof. Also, hindered phenols, hydroquinones, aromatic amines, and combinations thereof may be included. Exemplary plasticizers include, but are not limited to, lactams such as caprolactam and lauryl lactam, sulfonamides such as ortho- and para-toluenesulfonamide and N-ethyl, N-butyl benzylensulfonamide, and combinations thereof, as well as other plasticizers known in the art. Anti-blocking agent, for example, includes silica, diatomaceous earth, calcium carbonate, and talc.

In addition, other nucleating agents or clarifier may be used in combination with the nucleating agents of the present invention, for example organic acids, their metal salts or dibenzylidene sorbitol acetals (DBS). In general, the added amount of such additional nucleating agents or clarifiers is lower than usual, if they are used as only nucleating agent. Preferably no additional nucleating agent or clarifier is necessary in the polymer if a nucleating agent of formula (I) is used.

The methyl ester derivatives (formula (XI)) which are nucleating agents of formula (I) with R₃ = -C(O)CH₃ can be obtained by a single step reaction, wherein a suspension of a lactone of formula (X) and dimethylacetals of the formula (MeO)₂CH-R₁ and (MeO)₂CH-R₂ are reacted under acidic conditions (see reaction scheme 1, index "n" in scheme 1 is equal to 1). As acid concentrated hydrochloric acid may be used. For a better dilution of the formed participate an inert organic solvent, such as acetonitrile, may be added to the reaction mixture. The addition of organic solvent is also beneficial for better stirring and better availability of unreacted starting material. Diacetal derivatives of sugar acid methyl esters of formula (XI) are obtained. The product can be filtered off and purified. Mixed diacetals with different acetal residues R₁ and R₂ can be separated for example by column chromatography e.g. on silica with ethylacetate and hexane as eluents. Without bound to any theory, it is assumed that the formation of the first acetal group takes place before the cleavage of the lactone ring of the starting material of formula (X), whereas the subsequent formation of the second acetal group required lactone cleavage.

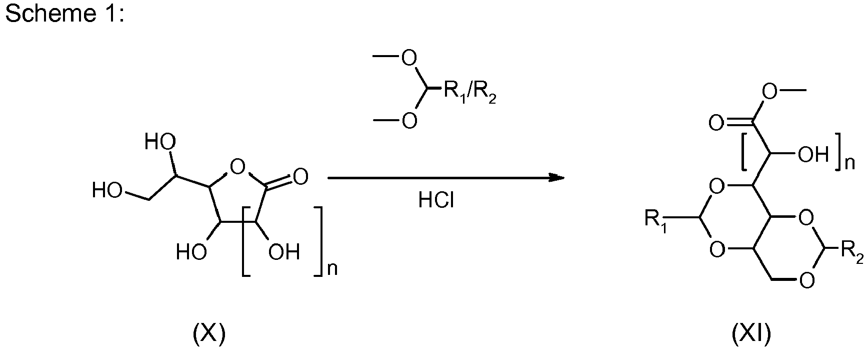

The meaning of n in formula (X) and (XI) is 1. Residues R₁ and R₂ in formula (XI) and in the corresponding dimethyl acetals are independently selected from substituted phenyl groups, wherein the substituents of said substituted phenyl groups are independently selected from the group consisting of (C₁-C₄)-alkyl-.

An example for the preparation method is the reaction of L-gulonic acid gamma lactone of formula (XII) with benzaldehyde dimethylacetals of formula (XIII) under acidic conditions to obtain 3,5:4,6-dibenzylidene L-gulonic acid methyl esters (XIV) according to scheme 2. 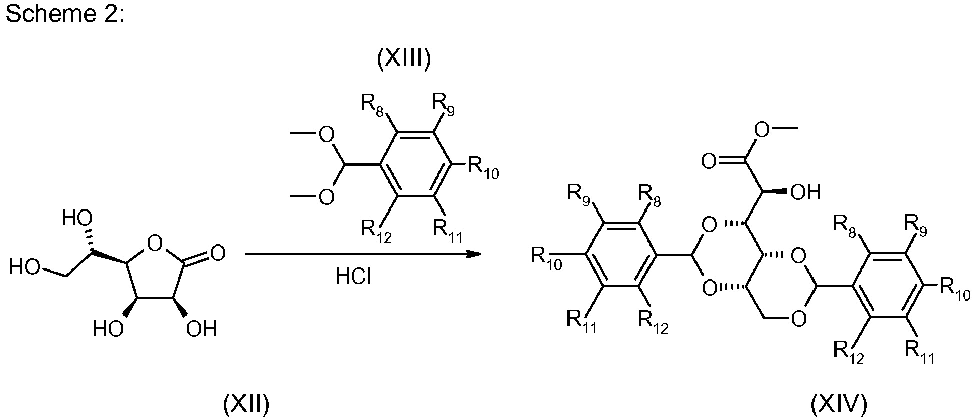 wherein
the substituents R₈, R₉, R₁₀, R₁₁ and R₁₂ of the phenyl groups in formula (XIV) or the corresponding benzylidene dimethyl acetals of formula (XIII) are independently from each other selected from the group consisting of hydrogen and (C₁-C₄)-alkyl-.

The obtained methyl esters of formula (XI) and (XIV) can be used as starting material for the preparation of the corresponding amides or substituted amides (compounds of formula (I) with R³ = -C(O)-NR₅R₆ (formula (III)), and hydrazine derivatives (compounds of formula (I) with R₃ = -C(O)-NR₇-NR₅R₆ (formula (IV)). The derivatization can be achieved simply by stirring a mixture of the methyl ester and the corresponding amine derivative or hydrazine derivative in methanol at elevated temperature. Preferred amine derivatives are ammonia, mono- or di-(C₁-C₄)-alkyl amines, hydrazine, mono-, di- or tri-(C₁-C₄)-alkyl hydrazines, in particular preferred are ammonia, methyl amine, ethyl amine, propyl amine, butyl amine, hydrazine, methyl hydrazine, ethyl hydrazine, propyl hydrazine, and butyl hydrazine.

Free acids (compounds of formula (I) with R₃ = -C(O)OH (formula (II)) are available from the corresponding methyl esters simply by stirring a suspension of the methyl ester in water in the presence of a base, preferably a metal hydroxide, such as potassium hydroxide or sodium hydroxide, at elevated temperature. The pH of the reaction mixture is adjusted between 1 and 6 with an acid, e. g. by addition of hydrochloric acid, to obtain the free acid form of the corresponding sugar acid diacetal.

The obtained diacetal sugar acid derivatives of the present invention may contain small amounts of by-products, such as monoacetals. The diacetal sugar acid derivatives may be used without further purification, but a removal of the impurities prior to incorporation of the compounds into the polymer may be desirable to improve the haze and clarity of the prepared polymer composition. The purification can be accomplished by extraction with a non-polar solvent, such as dialkyl ethers, for example. The purified products generally contain at least 90 wt.-% of the desired diacetal sugar acid derivatives of formula (I), preferably at least 95 wt.-%, most preferred 98 wt.-% or more.

The nucleating agents of the present invention may be added to a polymer to be nucleated by merely mixing the polymer composition with a nucleating agent of formula (I) by any suitable means, such as dry blending in a mechanical mixer. The composition may then be processed and fabricated by extrusion, molding, thermoforming, and the like into the desired fabricated article.

A further embodiment of the invention is the use of a nucleating agent of formula (I) as clarifier for polymer compositions, characterized in that at least one nucleating agent of formula (I) is blended with at least one polymer selected from olefinic homopolymers or olefinic copolymers, polystyrene, polyamides, polyesters, polyketones, polyoxyalkylenes, poly(phenylene)sulfide, polyvinylchloride or mixtures thereof.

The nucleating agents of formula (I) are in particularly useful as clarifier e.g. for the preparation of transparent polymer compositions, wherein the polymer composition comprises at least one polymer selected from the group consisting of olefinic homopolymers, olefinic copolymers, polystyrene, polyamides, polyesters, polyketones, polyoxyalkylenes, poly(phenylene)sulfide, polyvinylchloride. In particular the compounds of formula (Ia) are useful for this purpose. The compounds of formula (I) and (Ia) are for example suitable as clarifier for polymer compositions and/or for the preparation of transparent polymer compositions, wherein the polymer is selected from the group consisting of olefinic homopolymers derived from aliphatic olefins with 2 to 6 carbon atoms or olefinic copolymers derived from at least one aliphatic olefin with 2 to 6 carbon atoms and one or more ethylenically unsaturated comonomers, or wherein the polymer is a polyamide, such as polyamide-6,6, and polyamide-6 or mixtures thereof.

The polymer composition comprising nucleating agents of the present invention can be used for packaging materials and container materials for food, pharmaceutical compositions, cosmetics etc., in particular, for transparent packages or containers.

### Examples:

### Example 1: L-Gulonic acid gamma lactone

A solution of 23.1 g (0.13 mol) L-ascorbic acid and 0.1 g celite in 170.0 g water was hydrogenated over 3.0 g of 5 % Ru/Al₂O₃ in a hydrogenator at 50 °C, 30-40 bar hydrogen pressure and stirred with 1300 rpm for 6 h. After steady uptake of hydrogen, the reaction was cooled down and the catalyst was removed by filtration over celite. Water was removed by lyophilisation to yield 22.66 g (97 %) of a white fluffy powder, which was pure by NMR. On recrystallization of the sample from water, crystalline material was obtained.
m.p.: 186.62 °C; [α]_{D}⁹: + 55° (c= 4, H₂O); ¹H-NMR (400 MHz, DMSO-d₆): δ = 3.44-3.55 (m, 2, -CH₂); 3.71-3.77 (m, 1, -CH); 4.16-4.24 (m, 2, -CH); 4.43-4.45 (q, 1, -CH); 4.66 (t, 1, -OH); 4.97-4.98(d, 1, -OH); 5.33-5.34 (d, 1, -OH); 5.78-5.80 (d, 1, -OH). L-Gulonic acid gamma lactoneis commercially available, also.

### Examples 2-8:

### General procedure for examples 2-8

A round bottom flask, equipped with heating bath, condenser and a stirrer, was charged with trimethylorthoformiate (6.00 Eq.), dry methanol (500 mL/mol), concentrated hydrochloric acid (0.11 Eq.) and a substituted benzaldehyde (1.00 Eq.). The solution was stirred for approximately 24 h at 40 °C and then at room temperature until thin layer chromatography (TLC) showed full conversion of the starting material. A saturated sodium carbonate solution (50 mL/mol) based on benzaldehyde was added to the reaction mixture. The formed precipitate was filtered off and the liquid phase was extracted three times with petroleum benzene. The combined organic phases were dried over sodium sulfate and the organic layer was evaporated to yield the relevant substituted benzaldehyde dimethyl acetal.

### Example 2: 4-Methylbenzaldehyd dimethyl acetal

According to the general procedure 4-Methylbenzaldehyde (0.208 mol, 25.00 g) was treated with trimethylorthoformiate (1.248 mol, 136.53 mL) and concentrated hydrochloric acid (0.023 mol, 1.89 mL). General workup yielded the product as a clear liquid (31.1 g, 90 %). R_{f} = 0.80 (Ethylacetate - Hexane 1:5); ¹H-NMR (400 MHz, DMSO-d₆): δ = 2.30 (s, 3, -CH₃); 3.21 (s, 6, -OCH₃); 5.33 (s, 1, -OCHO-); 7.17-7.28 (m, 4, aromatic).

### Example 3: 3,4-Dimethylbenzaldehyd dimethyl acetal

According to the general procedure 3,4-Dimethylbenzaldehyde (0.186 mol, 25.00 g) was treated with trimethylorthoformiate (1.118 mol, 122.29 mL) and concentrated hydrochloric acid (0.021 mol, 1.79 mL). General workup yielded the product as a clear liquid (32.2 g, 96 %). R_{f} = 0.81 (Ethylacetate - Hexane 1:5); ¹H-NMR (400 MHz, DMSO-d₆): δ = 2.21 (s, 3, -CH₃); 2.22 (s, 3, -CH₃); 3.21 (s, 6, -OCH₃); 5.29 (s, 1, -OCHO-); 7.07-7.14 (m, 3, aromatic).

### Example 4: 3,5-Dimethylbenzaldehyd dimethyl acetal

According to the general procedure 3,5-Dimethylbenzaldehyde (0.186 mol, 25.00 g) was treated with trimethylorthoformiate (1.118 mol, 122.29 mL) and concentrated hydrochloric acid (0.021 mol, 1.70 mL). General workup yielded the product as a clear liquid (27.4 g, 82 %). R_{f} = 0.83 (Ethylacetate - Hexane 1:5); ¹H-NMR (400 MHz, DMSO-d₆): δ = 2.27 (s, 3, -CH₃); 2.27 (s, 3, -CH₃); 3.22 (s, 6, -OCH₃); 5.28 (s, 1, -OCHO-); 6.95-6.98 (m, 3, aromatic).

### Example 5: 2,4-Dimethylbenzaldehyd dimethyl acetal

According to the general procedure 2,4-Dimethylbenzaldehyde (0.186 mol, 25.00 g) was treated with trimethylorthoformiate (1.118 mol, 122.29 mL) and concentrated hydrochloric acid (0.21 mol, 1.70 mL). General workup yielded the product as a clear liquid (30.6 g, 91 %). R_{f} = 0.80 (Ethylacetate - Hexane 1:5); ¹H-NMR (400 MHz, DMSO-d₆): δ = 2.25 (s, 3, -CH₃); 2.26 (s, 3, -CH₃); 3.21 (s, 6, -OCH₃); 5.41 (s, 1, -OCHO-); 6.97-6.99 (t, 2, aromatic); 7.29-7.32 (d, 1, aromatic).

### Example 6: 2,5-Dimethylbenzaldehyd dimethyl acetal

According to the general procedure 2,5-Dimethylbenzaldehyde (0.186 mol, 25.00 g) was treated with trimethylorthoformiate (1.118 mol, 122.29 mL) and concentrated hydrochloric acid (0.021 mol, 1.70 mL). General workup yielded the product as a clear liquid (29.8 g, 89 %). R_{f} = 0.80 (Ethylacetate - Hexane 1:5); ¹H-NMR (400 MHz, DMSO-d₆): δ = 2.25 (s, 3, -CH₃); 2.27 (s, 3, -CH₃); 3.21 (s, 6, -OCH₃); 5.41 (s, 1, -OCHO-); 7.02-7.07 (m, 2, aromatic); 7.24 (s, 1, aromatic).

### Example 7: 4-Propylbenzaldehyd dimethyl acetal

According to the general procedure 4-Propylbenzaldehyde (0.169 mol, 25.00 g) was treated with trimethylorthoformiate (1.012 mol, 110.74 mL) and concentrated hydrochloric acid (0.019 mol, 1.55 mL). General workup yielded the product as a clear liquid (31.5 g, 96 %). R_{f} = 0.74 (Ethylacetate - Hexane 1:5); ¹H-NMR (400 MHz, DMSO-d₆): δ = 0.86-0.90 (t, 3, -CH₃); 1.54-1.63 (m, 2, -CH₂); 2.53-2.57 (t, 2, -CH₂); 3.22 (s, 6, -OCH₃); 5.34 (s, 1, -OCHO-); 7.17-7.19 (d, 2, aromatic); 7.28-7.29 (d, 2, aromatic).

### Example 8: 4-Isobutylbenzaldehyd dimethyl acetal

According to the general procedure 4-Isobutylbenzaldehyde (0.154 mol, 25.00 g) was treated with trimethylorthoformiate (0.925 mol, 101.15 mL) and concentrated hydrochloric acid (0.017 mol, 1.40 mL). General workup yielded the product as a clear liquid (28.2 g, 88 %). R_{f} = 0.78 (Ethylacetate - Hexane 1:5); ¹H-NMR (400 MHz, DMSO-d₆): δ = 0.84 (s, 3, -CH₃); 0.86 (s, 3, -CH₃); 1.77-1.87 (m, 1, -CH); 2.43-2.45 (d, 2, -CH₂); 3.22 (s, 6, -OCH₃); 5.34 (s, 1, -OCHO-); 7.14-7.16 (d, 2, aromatic); 7.27-7.29 (d, 2, aromatic).

### Examples 9-17:

### General procedure for examples 9-17

A round bottom flask, equipped with a mechanical stirrer, was charged with *L*-gulonic acid gamma lactone of Example 1 (1.00 Eq.); a benzaldehyde dialkyl acetal of the group of unsubstituted benzaldehyde dimethyl acetal, a substituted benzaldehyde dimethyl acetal of Example 2-8 and commercially available 4-Propylbenzaldehyd diethyl acetal (4.00 Eq.); and concentrated hydrochloric acid (0.40 Eq.). The suspension was stirred for about 24 h at room temperature. The formed participate is diluted with acetonitrile (725 mL/mol) based on *L*-Gulonic acid gamma lactone for better stirring and availability of unreacted starting material. The suspension is stirred until TLC showed full formation of the product. The product is filtered off and washed three times with water and diethyl ether each. The product is dried in vacuum at 110 °C to yield the corresponding 3,5:4,6-Dibenzylidene-*L*-gulonic acid methyl ester.

### Example 9: 3,5:4,6-Dibenzylidene-L-gulonic acid methyl ester

According to the general procedure *L*-gulonic acid gamma lactone (Example 1, 0.067 mol, 11.88 g) was reacted with commercial unsubstituted benzaldehyde dimethyl acetal(0.267 mol) and concentrated hydrochloric acid (0.027 mol, 2.21 mL). For better dilution 50.00 mL acetonitrile were added. General workup yielded the product as a white powder (22.72 g, 88 %). R_{f} = 0.70 (Ethylacetate - Hexane 4:1); ¹H-NMR (400 MHz, DMSO-d₆): δ = 3.63 (s, 3, -OCH₃); 4.02-4.30 (m, 6, -CH,-CH₂); 5.69 (s, 1, -OCHO-); 5.70 (s, 1, -OCHO-); 6.11-6.13 (d, *J* = 6.1 Hz, -OH); 7.36-7.48 (m, 10, aromatic); ¹³C-NMR (100 MHz, DMSO-d6): δ = 52.0 (1, -OCH₃); 68.1; 69.7; 70.2; 78.7 (5, -CH-, -CH₂-); 99.5; 99.7 (2, -OCHO-); 126.4; 126.5; 128.46; 128.49; 129.1; 129.2; 138.5; 139.0 (12, aromatic); 173.2 (1, -CO₂-); HRMS (EI): Calculated for C₂₁H₂₁O₇ [M-H]⁺ 385.1287; found 385.1282.

### Example 10: 3,5:4,6-Di-(4-Methylbenzylidene)-L-gulonic acid methyl ester

According to the general procedure *L*-gulonic acid gamma lactone (Example 1, 0.012 mol, 2.14 g) was reacted with the substituted benzaldehyde dimethyl acetal of Example 2 (0.048 mol, 8.09 mL) and concentrated hydrochloric acid (0.005 mol, 0.40 mL). For better dilution 10.00 mL acetonitrile were added. General workup yielded the product as a white powder (4.53 g, 91 %). R_{f} = 0.78 (Ethylacetate - Hexane 4:1); ¹H-NMR (400 MHz, DMSO-d₆): δ = 2.30 (s, 3, -CH₃); 2.31 (s, 3, -CH₃); 3.62 (s, 3, -OCH₃); 3.98-4.28 (m, 6, -CH,-CH₂); 5.63 (s, 1, -OCHO-); 5.64 (s, 1, -OCHO-); 6.08-6.10 (d, *J* = 6.1 Hz, -OH); 7.17-7.35 (m, 8, aromatic); ¹³C-NMR (100 MHz, DMSO-d₆): δ = 21.3 (2, -CH₃); 51.9 (1, -OCH₃); 68.1; 69.6; 70.1; 78.7 (5, -CH-, -CH₂-); 99.6; 99.8 (2, -OCHO-); 126.4; 126.5; 128.9; 129.0 (8, -CH-, aromatic); 135.7; 136.2; 138.3; 138.4 (12, aromatic); 173.3 (1, -CO₂-); HRMS (EI): Calculated for C₂₃H₂₅O₇ [M-H]⁺ 413.1600, found 413.1594.

### Example 11: 3,5:4,6-Bis-(3,4-Dimethylbenzylidene)-L-gulonic acid methyl ester

According to the general procedure *L*-gulonic acid gamma lactone (Example 1, 0.069 mol, 12.35 g) was reacted with the substituted benzaldehyde dimethyl acetal of Example 3 (0.277 mol, 50.71 mL) and concentrated hydrochloric acid (0.028 mol, 2.30 mL). For better dilution 50.00 mL acetonitrile were added. General workup yielded the product as a white powder (30.69 g, 93 %). R_{f} = 0.82 (Ethylacetate - Hexane 4:1); ¹H-NMR (400 MHz, DMSO-d₆): δ = 2.22 (s, 6, -CH₃); 2.23 (s, 6, -CH₃); 3.62 (s, 3, -OCH₃); 3.96-4.27 (m, 6, -CH,-CH₂); 5.60 (s, 2, -OCHO-); 6.07-6.08 (d, *J* = 6.1 Hz, -OH); 7.07-7.22 (m, 6, aromatic); ¹³C-NMR (100 MHz, DMSO-d₆): δ = 19.7; 19.9 (4, -CH₃); 51.9 (1, -OCH₃); 68.05; 68.11; 69.6; 70.2; 78.8 (5, -CH-, -CH₂-); 99.7; 99.9 (2, -OCHO-); 123.9; 124.0; 127.5; 127.6; 129.45; 129.47; 136.07; 136.11; 136.6; 136.97; 137.03 (12, aromatic); 173.3 (1, -CO₂-); HRMS (EI): Calculated for C₂₅H₂₉O₇ [M-H]⁺ 441.1913, found 441.1910.

### Example 12: 3,5:4,6-Bis-(3,5-Dimethylbenzylidene)-L-gulonic acid methyl ester

According to the general procedure *L*-gulonic acid gamma lactone (Example 1, 0.042 mol, 7.41 g) was reacted with the substituted benzaldehyde dimethyl acetal of Example 4 (0.152 mol, 27.79 mL) and concentrated hydrochloric acid (0.017 mol, 1.38 mL). For better dilution 30.00 mL acetonitrile were added. General workup yielded the product as a white powder (16.78 g, 91 %). R_{f} = 0.84 (Ethylacetate - Hexane 4:1); ¹H-NMR (400 MHz, DMSO-d₆): δ = 2.27 (s, 6, -CH₃); 2.28 (s, 6, -CH₃); 3.64 (s, 3, -OCH₃); 3.96-4.24 (m, 6, -CH,-CH₂); 5.60 (s, 2, -OCHO-); 6.09-6.10 (d, *J* = 6.1 Hz, -OH); 6.98; (s, 4, aromatic); 7.06 (s, 2, aromatic); ¹³C-NMR (100 MHz, DMSO-d₆): δ = 21.39; 21.41 (4, -CH₃); 51.9 (1, -OCH₃); 68.0; 68.2; 69.6; 70.2; 78.8 (5, -CH-, -CH₂-); 99.7; 100.0 (2, -OCHO-); 124.2; 124.3; 130.4; 137.4; 138.4; 138.9 (12, aromatic); 173.4 (1, -CO₂-); HRMS (EI): Calculated for C₂₅H₂₉O₇ [M-H]⁺ 441.1913, found 441.1907.

### Example 13: 3,5:4,6-Bis-(2,4-Dimethylbenzylidene)-L-gulonic acid methyl ester

According to the general procedure *L*-gulonic acid gamma lactone (Example 1, 0.042 mol, 7.41 g) was reacted with the substituted benzaldehyde dimethyl acetal of Example 5 (0.166 mol, 30.43 mL) and concentrated hydrochloric acid (0.017 mol, 1.38 mL). For better dilution 30.00 mL acetonitrile were added. General workup yielded the product as a white powder (16.56 g, 90 %). R_{f} = 0.81 (Ethylacetate - Hexane 4:1); ¹H-NMR (400 MHz, DMSO-d₆): δ = 2.23 (s, 3, CH₃); 2.25-2.26 (d, 6, -CH₃); 2.34 (s, 3, -CH₃); 3.57 (s, 3, -OCH₃); 3.97-4.20 (m, 6, -CH,-CH₂); 5.71 (d, 2, -OCHO-); 6.01-6.03 (d, *J* = 6.0 Hz, -OH); 6.97-7.00 (t, 4, aromatic); 7.34-7.38 (t, 2, aromatic); ¹³C-NMR (100 MHz, DMSO-d₆): δ = 18.7; 21.2 (4, -CH₃); 51.9 (1, -OCH₃); 68.1; 68.2; 69.7; 70.1; 78.8 (5, -CH-, -CH₂-); 98.6; 99.4 (2, -OCHO-); 126.21; 126.23; 126.3; 126.8; 131.3; 131.4; 133.6; 134.1; 136.3; 136.4; 138.1; 138.2 (12, aromatic); 173.3 (1, -CO₂-); HRMS (EI): Calculated for C₂₅H₂₉O₇ [M-H]⁺ 441.1913, found 441.1906.

### Example 14: 3,5:4,6-Bis-(2,5-Dimethylbenzylidene)-L-gulonic acid methyl ester

According to the general procedure *L*-gulonic acid gamma lactone (Example 1, 0.041 mol, 7.36 g) was reacted with the substituted benzaldehyde dimethyl acetal of Example 6 (0.165 mol, 30.20 mL) and concentrated hydrochloric acid (0.017 mol, 1.37 mL). For better dilution 30.00 mL acetonitrile were added. General workup yielded the product as a white powder (15.00 g, 82 %). R_{f} = 0.81 (Ethylacetate - Hexane 4:1); ¹H-NMR (400 MHz, DMSO-d₆): δ = 2.23 (s, 3, -CH₃); 2.27 (s, 6, -CH₃); 2.34 (s, 3, -CH₃); 3.58 (s, 3, -OCH₃); 4.00-4.22 (m, 6, -CH,-CH₂); 5.73 (s, 1, -OCHO-); 5.74 (s, 1, -OCHO-); 6.03-6.04 (d, *J* = 6.0 Hz, -OH); 7.05-7.07 (d, 4, aromatic); 7.31-7.33 (d, 2, aromatic); ¹³C-NMR (100 MHz, DMSO-d₆): δ = 18.3; 21.2 (4, -CH₃); 51.9 (1, -OCH₃); 68.2; 69.7; 70.2; 78.8 (5, -CH-, -CH₂-); 98.6; 99.4 (2, -OCHO-); 126.9; 127.4; 129.5; 130.6; 130.7; 133.3; 133.4; 134.55; 134.57; 136.1; 136.6 (12, aromatic); 173.3 (1, -CO₂-); HRMS (EI): Calculated for C₂₅H₂₉O₇ [M-H]⁺ 441.1913, found 441.1909.

### Example 15: 3,5:4,6-Di-(4-Propylbenzylidene)-L-gulonic acid methyl ester

According to the general procedure *L*-gulonic acid gamma lactone (Example 1, 0.013 mol, 2.24 g) was reacted with the substituted benzaldehyde dimethyl acetal of Example 7 (0.050 mol, 9.89 mL) and concentrated hydrochloric acid (0.005 mol, 0.42 mL). For better dilution 10.00 mL acetonitrile were added. General workup yielded the product as a white powder (4.91 g, 81 %). R_{f} = 0.83 (Ethylacetate - Hexane 4:1); ¹H-NMR (400 MHz, DMSO-d₆): δ = 0.85-0.90 (m, 6, -CH₃); 1.52-1.62 (m, 4, -CH₂-); 2.52-2.57 (m, 4, -CH₂-); 3.62 (s, 3, -OCH₃); 3.98-4.28 (m, 6, -CH,-CH₂); 5.64 (s, 1, -OCHO-); 5.65 (s, 1, -OCHO-); 6.09-6.10 (d, *J* = 6.1 Hz, -OH); 7.17-7.21 (t, 4, aromatic); 7.27-7.29 (d, 2, aromatic); 7.36-7.38 (d, 2, aromatic); ¹³C-NMR (100 MHz, DMSO-d₆): δ = 14.1 (2, -CH₃); 24.5; 24.6 (2, -CH₂-); 37.5; 37.6 (2, -CH₂-); 52.0 (1, -OCH₃); 68.06; 68.09; 69.7; 70.1; 78.7 (5, -CH-, -CH₂-); 99.6; 99.8 (2, -OCHO-); 126.3; 126.5; 128.4; 136.0; 136.5; 143.0; 143.1 (12, aromatic); 173.2 (1, -CO₂-); HRMS (EI): Calculated for C₂₇H₃₃O₇ [M-H]⁺ 469.2226, found 469.2216.

### Example 16: 3,5:4,6-Di-(4-Isobutylbenzylidene)-L-gulonic acid methyl ester

According to the general procedure *L*-gulonic acid gamma lactone (Example 1, 0.034 mol, 6.02 g) was reacted with the substituted benzaldehyde dimethyl acetal of Example 8 (0.135 mol, 28.56 mL) and concentrated hydrochloric acid (0.014 mol, 1.12 mL). For better dilution 25.00 mL acetonitrile were added. General workup yielded the product as a white powder (9.88 g, 59 %). R_{f} = 0.85 (Ethylacetate - Hexane 4:1); ¹H-NMR (400 MHz, DMSO-d₆): δ = 0.84 (d, 6, -CH₃); 0.86 (d, 6 -CH₃); 1.78-1.86 (m, 2, -CH-); 2.43-2.46 (q, 4, -CH₂-); 3.63 (s, 3, -OCH₃); 3.99-4.29 (m, 6, -CH,-CH₂); 5.65 (s, 1, -OCHO-); 5.66 (s, 1, -OCHO-); 6.09-6.11 (d, *J* = 6.1 Hz, -OH); 7.14-7.18 (t, 4, aromatic); 7.28-7.29 (d, 2, aromatic); 7.36-7.38 (d, 2, aromatic); ¹³C-NMR (100 MHz, DMSO-d₆): δ = 22.6 (4, -CH₃); 30.1 (2, -CH-); 44.81; 44.83 (2, -CH₂-); 52.0 (1, -OCH₃); 68.1; 69.7; 70.2; 78.7 (5, -CH-, -CH₂-); 99.6; 99.8 (2, -OCHO-); 126.2; 126.3; 129.0; 136.0; 136.5; 142.0; 142.1 (12, aromatic); 173.2 (1, -CO₂-); HRMS (EI): Calculated for C₂₉H₃₇O₇ [M-H]⁺ 497.2539, found 497.2532.

### Example 17: 3,5:4,6-Di-(4-Propylbenzylidene)-L-gulonic acid ethyl ester

According to the general procedure *L*-gulonic acid gamma lactone (Example 1, 0.021 mol, 3.75 g) was reacted with the commercial 4-Propylbenzaldehyd diethyl acetal (0.084 mol, 20.00 mL) and concentrated hydrochloric acid (0.008 mol, 0.70 mL). For better dilution 15.00 mL acetonitrile were added. General workup yielded the product as a white powder (8.21 g, 81 %). R_{f} = 0.85 (Ethylacetate - Hexane 4:1); ¹H-NMR (400 MHz, DMSO-d₆): δ = 0.85-0.90 (q, 6, -CH₃); 1.11-1.15 (t, 3, -OCH₂-CH₃) 1.52-1.62 (m, 4, -CH₂-); 2.52-2.57 (m, 4, -CH₂-); 3.98-4.25 (m, 8, -CH,-CH₂, -OCH₂-); 5.64 (s, 1, -OCHO-); 5.65 (s, 1, -OCHO-); 6.04-6.05 (d, *J* = 6.0 Hz, -OH); 7.17-7.21 (t, 4, aromatic); 7.28-7.30 (d, 2, aromatic); 7.36-7.38 (d, 2, aromatic); ¹³C-NMR (100 MHz, DMSO-d₆): δ = 14.0; 14.1; 14.5 (3, -CH₃, -OCH₂-CH₃); 24.5; 24.6 (2, -CH₂-); 37.4; 37.5 (2, -CH₂-); 60.5; 68.09; 68.12; 69.7; 70.1; 78.8 (5, -CH-, -CH₂-, -OCH₂-); 99.5; 99.8 (2, -OCHO-); 126.3; 126.5; 128.3; 128.4; 136.0; 136.5; 143.0; 143.1 (12, aromatic); 172.8 (1, -CO₂-); HRMS (EI): Calculated for C₂₈H₃₅O₇ [M-H]⁺ 483.2383, found 483.2375.

### Examples 18:25:

### General procedure for examples 18-25

A round bottom flask, equipped with a heating bath, condenser and a stirrer, was charged with a 3,5:4,6-Dibenzylidene-*L*-gulonic acid methyl ester of Example 9-17) (1.00 Eq.), potassium hydroxide (4.00 Eq.) and water (5.13 L/mol) based on Example the corresponding 3,5:4,6-Dibenzylidene-*L*-gulonic acid methyl ester. The suspension was stirred for about 16 h at 80 °C and thereafter at room temperature until TLC showed full formation of the product. Hydrochloric acid (2 N) was added until pH 2 is achieved. The product was filtered off and washed three times with water. The product was dried in vacuum at 110 °C to yield the corresponding substituted 3,5:4,6-Dibenzylidene-*L*-gulonic acid.

### Example 18: 3,5:4,6-Dibenzylidene-L-gulonic acid

According to the general procedure the 3,5:4,6-Dibenzylidene-*L*-gulonic acid methyl ester of Example 9 (0.013 mol, 5.00 g) was treated with KOH (0.065 mol, 3.63 g) in 65.00 mL water. General workup yielded the product as a white powder (4.45 g, 92 %). R_{f} = 0.11 (Ethylacetate - Hexane 4:1); ¹H-NMR (400 MHz, DMSO-d₆): δ = 4.00-4.23 (m, 6, -CH,-CH₂); 5.69 (s, 1, -OCHO-); 5.70 (s, 1, -OCHO-); 7.36-7.49 (m, 10, aromatic); ¹³C-NMR (100 MHz, DMSO-d₆): δ = 68.2; 69.7; 70.2; 78.9 (5, -CH-, -CH₂-); 99.7 (2, -OCHO-); 126.6; 128.5; 129.1; 129.2; 138.6; 139.0 (12, aromatic); 173.2 (1, -CO₂-); HRMS (EI): Calculated for C₂₆H₃₅O₇Si₂ [M-H]⁺ 515.1921, found 515.1918.

### Example 19: 3,5:4,6-Di-(4-Methylbenzylidene)-L-gulonic acid

According to the general procedure the 3,5:4,6-Dibenzylidene-*L*-gulonic acid methyl ester of Example 10 (0.012 mol, 5.00 g) was treated with KOH (0.060 mol, 3.38 g) in 60.00 mL water. General workup yielded the product as a white powder (4.55 g, 94 %). R_{f} = 0.03 (Ethylacetate - Hexane 4:1); ¹H-NMR (400 MHz, DMSO-d₆): δ = 2.30 (s, 3, -CH₃); 2.31 (s, 3, -CH₃); 3.96-4.20 (m, 6, -CH,-CH₂); 5.63 (s, 2, -OCHO-); 7.16-7.36 (m, 8, aromatic); ¹³C-NMR (100 MHz, DMSO-d₆): δ = 21.3 (2, -CH₃); 68.2; 69.7; 70.2; 78.9 (5, -CH-, -CH₂-); 99.8 (2, -OCHO-); 126.49; 126.53; 128.92; 128.93; 135.8; 136.3; 138.3; 138.4 (12, aromatic); 174.3 (1, -CO₂-); HRMS (EI): Calculated for C₂₈H₃₉O₇Si₂ [M-H]⁺ 543.2234, found 543.2228.

### Example 20: 3,5:4,6-Bis-(3,4-Dimethylbenzylidene)-L-gulonic acid

According to the general procedure the 3,5:4,6-Dibenzylidene-*L*-gulonic acid methyl ester of Example 11 (0.011 mol, 5.00 g) was treated with KOH (0.057 mol, 3.17 g) in 55.00 mL water. General workup yielded the product as a white powder (4.80 g, 99 %). R_{f} = 0.08 (Ethylacetate - Hexane 4:1); ¹H-NMR (400 MHz, DMSO-d₆): δ = 2.21 (s, 6, -CH₃); 2.23 (s, 6, -CH₃); 3.86-4.15 (m, 6, -CH,-CH₂); 5.55 (s, 1, -OCHO-); 5.56 (s, 1, -OCHO); 7.10-7.22 (m, 6, aromatic); ¹³C-NMR (100 MHz, DMSO-d₆): δ = 19.7; 19.88; 19.90 (4, -CH₃); 68.7; 68.8; 69.7; 70.3; 79.3 (5, -CH-, -CH₂-); 99.9; 100.0 (2, -OCHO-); 124.0; 124.2; 127.6; 127.7; 129.4; 129.4; 136.0; 136.1; 136.4; 136.7; 136.9; 137.0 (12, aromatic); 174.5 (1, -CO₂-); HRMS (EI): Calculated for C₃₀H₄₃O₇Si₂ [M-H]⁺ 571.2547, found 571.2551.

### Example 21: 3,5:4,6-Bis-(3,5-Dimethylbenzylidene)-L-gulonic acid

According to the general procedure the 3,5:4,6-Dibenzylidene-*L*-gulonic acid methyl ester of Example 12 (0.011 mol, 5.00 g) was treated with KOH (0.057 mol, 3.17 g) in 55.00 mL water. General workup yielded the product as a white powder (4.79 g, 99 %). R_{f} = 0.11 (Ethylacetate - Hexane 4:1); ¹H-NMR (400 MHz, DMSO-d₆): δ = 2.27 (s, 6, -CH₃); 2.29 (s, 6, -CH₃); 3.91-4.17 (m, 6, -CH,-CH₂); 5.58 (s, 2, -OCHO-); 6.99-7.08 (s, 6, aromatic); ¹³C-NMR (100 MHz, DMSO-d₆): δ = 21.4 (4, -CH₃); 68.4; 69.6; 70.3; 79.1 (5, -CH-, -CH₂-); 100.0 (2, -OCHO-); 124.3; 124.4; 130.4; 137.4; 138.6; 139.0 (12, aromatic); 174.4 (1, -CO₂-); HRMS (EI): Calculated for C₃₀H₄₃O₇Si₂ [M-H]⁺ 571.2547, found 571.2548.

### Example 22: 3,5:4,6-Bis-(2,4-Dimethylbenzylidene)-L-gulonic acid

According to the general procedure the 3,5:4,6-Dibenzylidene-*L*-gulonic acid methyl ester of Example 13 (0.011 mol, 5.00 g) was treated with KOH (0.057 mol, 3.17 g) in 55.00 mL water. General workup yielded the product as a white powder (4.44 g, 92 %). R_{f} = 0.09 (Ethylacetate - Hexane 4:1); ¹H-NMR (400 MHz, DMSO-d₆): δ = 2.25-2.28 (d, 9, -CH₃); 2.34 (s, 3, -CH₃); 3.95-4.19 (m, 6, -CH,-CH₂); 5.70 (s, 2, -OCHO-); 6.97-6.99 (d, 4, aromatic); 7.36-7.38 (d, 2, aromatic); ¹³C-NMR (100 MHz, DMSO-d₆): δ = 18.9; 19.0; 21.2 (4, -CH₃); 68.2; 68.3; 69.8; 70.2; 79.0 (5, -CH-, -CH₂-); 98.9; 99.3 (2, -OCHO-); 126.1; 126.2; 126.5; 126.7; 131.3; 131.4; 133.7; 134.2; 136.4; 136.5; 138.1 (12, aromatic); 174.4 (1, -CO₂-); HRMS (EI): Calculated for C₃₀H₄₃O₇Si₂ [M-H]⁺ 571.2547, found 571.2550.

### Example 23: 3,5:4,6-Bis-(2,5-Dimethylbenzylidene)-L-gulonic acid

According to the general procedure the 3,5:4,6-Dibenzylidene-*L*-gulonic acid methyl ester of Example 14 (0.011 mol, 5.00 g) was treated with KOH (0.057 mol, 3.17 g) in 55.00 mL water. General workup yielded the product as a white powder (4.71 g, 97 %). R_{f} = 0.09 (Ethylacetate - Hexane 4:1); ¹H-NMR (400 MHz, DMSO-d₆): δ = 2.27 (s, 6, -CH₃); 2.30 (s, 3, -CH₃); 2.34 (s, 3, -CH₃); 3.74-4.15 (m, 6, -CH, -CH₂); 5.64 (s, 1, -OCHO-); 5.66 (s, 1, -OCHO-); 7.03-7.05 (d, 4, aromatic); 7.33 (d, 2, aromatic); ¹³C-NMR (100 MHz, DMSO-d₆): δ =18.6; 18.7; 21.1 (4, -CH₃); 69.3; 69.4; 69.9; 70.5; 80.0 (5, -CH-, -CH₂-); 99.1; 99.2 (2, -OCHO-); 127.3; 127.4; 129.3; 130.5; 130.6; 133.4; 133.5; 134.3; 134.4; 136.6; 136.8 (12, aromatic); 174.5 (1, -CO₂-); HRMS (EI): Calculated for C₃₀H₄₃O₇Si₂ [M-H]⁺ 571.2547, found 571.2553.

### Example 24: 3,5:4,6-Di-(4-Propylbenzylidene)-L-gulonic acid

According to the general procedure the 3,5:4,6-Dibenzylidene-*L*-gulonic acid methyl ester of Example 15 (0.010 mol, 5.00 g) was treated with KOH (0.052 mol, 2.89 g) in 50.00 mL water. General workup yielded the product as a white powder (4.70 g, 100 %). R_{f} = 0.09 (Ethylacetate - Hexane 4:1); ¹H-NMR (400 MHz, DMSO-d₆): δ = 0.37 (t, 6, -CH₃); 1.55-1.60 (q, 4, -CH₂-); 2.54 (t, 4, -CH₂-); 3.84-4.14 (m, 6, -CH-, -CH₂-); 5.58 (s, 2, -OCHO-); 7.16-7.20 (t, 4, aromatic); 7.33-7.37 (t, 4, aromatic); ¹³C-NMR (100 MHz, DMSO-d₆): δ = 14.0; 14.1 (2, -CH₃); 24.56; 24.59 (2, -CH₂-); 37.5 (2, -CH₂-); 69.1; 69.3; 69.8; 70.4; 80.0 (5, -CH-, -CH₂-); 99.8; 99.9 (2, -OCHO-); 126.5; 126.7; 128.25; 128.30; 136.4; 136.7; 142.9; 143.0 (12, aromatic); 174.6 (1, -CO₂-); HRMS (EI): Calculated for C₃₂H₄₇O₇Si₂ [M-H]⁺ 599.2860, found 599.2868.

### Example 25: 3,5:4,6-Di-(4-Isobutylbenzylidene)-L-gulonic acid

According to the general procedure the 3,5:4,6-Dibenzylidene-*L*-gulonic acid methyl ester of Example 16 (0.010 mol, 5.00 g) was treated with KOH (0.050 mol, 2.81 g) in 50.00 g water. General workup yielded the product as a white powder (4.62 g, 95 %). R_{f} = 0.19 (Ethylacetate - Hexane 4:1); ¹H-NMR (400 MHz, DMSO-d₆): δ = 0.84-0.86 (d, 12, -CH3); 1.79-1.85 (quint, 2, -CH-); 2.44-2.45 (d, 4, -CH₂-); 3.85-4.15 (m, 6, -CH-, -CH₂-); 5.59 (s, 2, -OCHO-); 7.13-7.16 (t, 4, aromatic); 7.34-7.37 (t, 4, aromatic); ¹³C-NMR (100 MHz, DMSO-d₆): δ = 22.6 (4, -CH₃); 30.11; 30.13 (2, -CH-); 44.9 (2, -CH₂-); 69.3; 69.8; 70.5; 79.7 (5, -CH-, -CH₂-); 99.8; 99.9 (2, -OCHO-); 126.3; 126.6; 128.8; 128.9; 136.5; 136.7; 141.8; 141.9 (12, aromatic); 174.4 (1, -CO₂-); HRMS (EI): Calculated for C₃₄H₅₁O₇Si₂ [M-H]⁺ 627.3173, found 627.3186.

### Examples 26-33:

### General procedure for examples 26-33

A beaded rim flask, equipped with aluminum heating block, magnetic stirrer and pressure seal was charged with a 3,5:4,6-Dibenzylidene-*L*-gluconic acid methyl ester of Example 9-14 and 16-17 (1.00 Eq.) and ammonia in methanol (2 N) (7 Eq.). The suspension was stirred for about 16 h at 70 °C and overnight at room temperature until TLC showed full conversion. The formed powder was isolated by filtration and washed three times with diethyl ether. The product was dried in a vacuum at 110 °C to yield the corresponding 3,5:4,6-Dibenzylidene-*L*-gulonic amide.

### Example 26: 3,5:4,6-Dibenzylidene-L-gulonic amide

According to the general procedure the 3,5:4,6-Dibenzylidene-*L*-gulonic acid methyl ester of Example 9 (0.026 mol, 10.00 g) was reacted with ammonia in methanol (0.776 mol, 110.92 mL). General workup yielded the product as a white powder (9.37 g, 98 %). R_{f} = 0.40 (Ethylacetate - Hexane 4:1); ¹H-NMR (400 MHz, DMSO-d₆): δ = 3.98-4.23 (m, 6, -CH-, -CH₃-); 5.31-5.33 (d, 2, J = 5.3 Hz, -OH), 5.66 (s, 1, -OCHO-); 5.68 (s, 1, -OCHO-); 6.99 (bs, 1, -NH₂); 7.36-7.50 (m, 11, -NH₂, aromatic); ¹³C-NMR (100 MHz, DMSO-d₆): δ = 68.3; 68.8; 69.8; 70.4; 79.0 (5, -CH, -CH₂-); 99.7; 99.9 (2, -OCHO-); 126.7; 128.4; 129.1; 129.1; 138.7; 139.1 (12, aromatic); 174.2 (1, -CON-); HRMS (EI): Calculated for C₂₀H₂₀NO₆ [M-H]⁺ 370.1291, found 370.1282.

### Example 27: 3,5:4,6-Di-(4-Methylbenzylidene)-L-gulonic amide

According to the general procedure the 3,5:4,6-Dibenzylidene-*L*-gulonic acid methyl ester of Example 10 (0.012 mol, 5.00 g) was reacted with ammonia in methanol (0.362 mol, 51.70 mL). General workup yielded the product as a white powder (4.55 g, 94 %). R_{f} = 0.38 (Ethylacetate - Hexane 4:1); ¹H-NMR (400 MHz, DMSO-d₆): δ = 2.30-2.31 (d, 6, -CH₃); 3.94-4.20 (m, 6, -CH-, -CH₂-); 5.27-5.29 (d, J = 5.3 Hz, 1, -OH); 5.61-5.63 (d, 2, -OCHO-); 6.99 (bs, 1, -NH₂); 7.16-7.37 (m, 8, aromatic); 7.43 (bs, 1, -NH₂); ¹³C-NMR (100 MHz, DMSO-d₆): δ = 21.3 (2, -CH₃); 68.3; 68.8; 69.7; 70.4; 79.0 (5, -CH-, -CH₂-); 99.8; 99.9; (2, -OCHO-); 126.6; 128.9; 136.0; 136.3; 138.3 (12, aromatic); 174.3 (1, -CON-); HRMS (EI): Calculated for C₂₂H₂₄NO₆ [M-H]⁺ 398.1604, found 398.1596.

### Example 28: 3,5:4,6-Bis-(3,4-Dimethylbenzylidene)-L-gulonic amide

According to the general procedure the 3,5:4,6-Dibenzylidene-*L*-gulonic acid methyl ester of Example 11 (0.094 mol, 41.78 g) was reacted with ammonia in methanol (3.493 mol, 0.5 L). General workup yielded the product as a white powder (39.55 g, 98 %). R_{f} = 0.43 (Ethylacetate - Hexane 4:1); ¹H-NMR (400 MHz, DMSO-d₆): δ = 2.22-2.23 (bd, 12, -CH₃); 3.92-4.18 (m, 6, -CH-, -CH₂-); 5.26-5.28 (d, J = 5.3 Hz, 1, -OH); 5.57 (s, 1, -OCHO-); 5.59 (s, 1, -OCHO-); 6.99 (bs, 1, -NH₂); 7.10-7.24 (m, 6, aromatic); 7.43 (bs, 1, -NH₂); ¹³C-NMR (100 MHz, DMSO-d₆): δ = 19.7; 19.9 (4, -CH₃); 68.3; 68.8; 69.7; 70.4; 79.1 (5, -CH-, -CH₂-); 99.9; 100.1 (2, -OCHO-); 124.2; 127.8; 129.3; 129.4; 136.0; 136.3; 136.6; 136.9; 137.0 (12, aromatic); 174.3 (1, -CON-); HRMS (EI): Calculated for C₂₄H₂₈NO₆ [M-H]⁺ 426.1917, found 426.1913.

### Example 29: 3,5:4,6-Bis-(3,5-Dimethylbenzylidene)-L-gulonic amide

According to the general procedure the 3,5:4,6-Dibenzylidene-*L*-gulonic acid methyl ester of Example 12 (0.016 mol, 7.00 g) was reacted with ammonia in methanol (0.475 mol, 67.80 mL). General workup yielded the product as a white powder (6.37 g, 94 %). R_{f} = 0.58 (Ethylacetate - Hexane 4:1); ¹H-NMR (400 MHz, DMSO-d₆): δ = 2.27-2-28 (d, 12, -CH₃); 3.92-4.18 (m, 6, -CH-, -CH₂-); 5.29-5.31 (d, J = 5.3 Hz, 1, -OH); 5.56 (s, 1, -OCHO-); 5.58 (s, 1, -OCHO-); 6.98-7.08 (m, 7, -NH₂, aromatic); 7.46 (bs, 1, -NH₂); ¹³C-NMR (100 MHz, DMSO-d₆): δ = 21.4 (4, -CH₃); 68.3; 68.8; 69.7; 70.4; 79.1 (5, -CH-; -CH₂-); 100.1; 100.1 (2, -OCHO-); 124.5; 124.6; 130.3; 130.4; 137.30; 137.32; 138.7; 139.0 (12, aromatic); 174.3 (1, -CON-); HRMS (EI): Calculated for C₂₄H₂₈NO₆ [M-H]⁺ 426.1917, found 426.1913.

### Example 30: 3,5:4,6-Bis-(2,4-Dimethylbenzylidene)-L-gulonic amide

According to the general procedure the 3,5:4,6-Dibenzylidene-*L*-gulonic acid methyl ester of Example 13 (0.016 mol, 7.00 g) was reacted with ammonia in methanol (0.475 mol, 67.80 mL). General workup yielded the product as a white powder (6.56 g, 97 %). R_{f} = 0.49 (Ethylacetate - Hexane 4:1); ¹H-NMR (400 MHz, DMSO-d₆): δ = 2.25-2.26 (d, 6, -CH₃); 2.28 (s, 3, -CH₃); 2.35 (s, 3, -CH₃); 3.93-4.20 (m, 6, -CH-,-CH₂-); 5.18-5.19 (d, J = 5.3 Hz, 1, -OH); 5.66 (s, 1, -OCHO-); 5.70 (s, 1, - OCHO-); 6.95-6.98 (m, 5, -NH₂, aromatic); 7.37-7.41 (t, 3, -NH₂, aromatic); ¹³C-NMR (100 MHz, DMSO-d₆): δ = 19.0; 19.1; 21.2 (4, -CH₃); 68.3; 68.8; 69.9; 70.4; 79.1 (5, -CH-, -CH₂-); 98.9; 99.2 (2, -OCHO-); 126.07; 126.11; 126.5; 126.6; 131.26; 131.30; 133.9; 134.2; 136.49; 136.51; 138.00; 138.02 (12, aromatic); 174.4 (1, -CON-); HRMS (EI): Calculated for C₂₄H₂₈NO₆ [M-H]⁺ 426.1917, found 426.1914.

### Example 31: 3,5:4,6-Bis-(2,4-Dimethylbenzylidene)-L-gulonic amide

According to the general procedure the 3,5:4,6-Dibenzylidene-*L*-gulonic acid methyl ester of Example 14 (0.011 mol, 5.00 g) was reacted with ammonia in methanol (0.339 mol, 48.43 mL). General workup yielded the product as a white powder (4.67 g, 97 %). R_{f} = 0.49 (Ethylacetate - Hexane 4:1); ¹H-NMR (400 MHz, DMSO-d₆): δ = 2.26-2.27 (d, 9, -CH₃); 2.35 (s, 3, -CH₃); 3.95-4.22 (m, 6, -CH-, -CH₂-); 5.20-5.22 (d, J = 5.3 Hz, 1, -OH); 5.68 (s, 1, -OCHO-); 5.71 (s, 1, -OCHO-); 6.95 (bs, 1, -NH₂); 7.04-7.05 (d, 4, aromatic); 7.33-7.36 (d, 2, aromatic); 7.39 (bs, 1, -NH₂); ¹³C-NMR (100 MHz, DMSO-d₆): δ = 18.6; 18.7; 21.1; 21.2 (4, -CH₃); 68.3; 68.8; 69.8; 70.5; 79.1 (5, -CH-, -CH₂-); 98.9; 99.2 (2, -OCHO-); 127.2; 127.3; 129.35; 129.38; 130.5; 130.6; 133.4; 133.5; 134.4; 134.5; 136.4; 136.7 (12, aromatic); 174.4 (1, -CON-); HRMS (EI): Calculated for C₂₄H₂₉NO₆ [M]⁺ 427.1995, found 427.1989.

### Example 32: 3,5:4,6-Di-(4-Propylbenzylidene)-L-gulonic amide

According to the general procedure the 3,5:4,6-Dibenzylidene-*L*-gulonic acid ethyl ester of Example 17 (0.017 mol, 8.21 g) was reacted with ammonia in methanol (0.508 mol, 72.61 mL). General workup yielded the product 7h as a white powder (6.89 g, 89 %). R_{f} = 0.54 (Ethylacetate - Hexane 4:1); ¹H-NMR (400 MHz, DMSO-d₆): δ = 0.85-0.90 (t, 6, -CH₃); 1.53-1.62 (m, 4, -CH₂-); 2.53-2.57 (t, 4, -CH₂-); 3.94-4.20 (m, 6, -CH-, -CH₂-); 5.27-5.29 (d, J = 5.3 Hz, 1, -OH); 5.61 (s, 1, -OCHO-); 5.63 (s, 1, -OCHO-); 6.97 (bs, 1, -NH₂); 7.17-7.20 (t, 4, aromatic); 7.33-7.39 (m, 4, aromatic); 7.43 (bs, 1, -NH₂); ¹³C-NMR (100 MHz, DMSO-d₆): δ = 14.03; 14.04 (2, -CH₃); 24.56; 24.58 (2, -CH₂-); 37.5 (2, -CH₂-); 68.3; 68.8; 69.8; 70.4; 79.0 (5, -CH-, -CH₂-); 99.8; 100.0 (2, -OCHO-); 126.6; 128.3; 136.2; 136.6; 142.98; 143.04 (12, aromatic); 174.3 (1, -CON-); HRMS (EI): Calculated for C₂₆H₃₂NO₆ [M-H]⁺ 454.2230, found 454.2224.

### Example 33: 3,5:4,6-Di-(4-Isobutylbenzylidene)-L-gulonic amide

According to the general procedure the 3,5:4,6-Dibenzylidene-*L*-gulonic acid methyl ester of Example 16 (0.009 mol, 4.50 g) was reacted with ammonia in methanol (0.271 mol, 38.68 mL). General workup yielded the product 7i as a white powder (4.09 g, 94 %). R_{f} = 0.61 (Ethylacetate - Hexane 4:1); ¹H-NMR (400 MHz, DMSO-d₆): δ = 0.84-0.86 (dd, 12, -CH₃); 1.78-1.86 (m, 2, -CH-); 2.43-2.46 (q, 4, -CH₂-); 3.94-4.20 (m, 6, -CH-, -CH₂-); 5.28-5.30 (d, J = 5.3 Hz, 1, -OH); 5.62 (s, 1, -OCHO-); 5.64 (s, 1, -OCHO-); 6.98 (bs, 1, -NH₂); 7.15 (t, 4, aromatic); 7.34-7.39 (m, 4, aromatic); 7.44 (bs, 1, -NH₂); ¹³C-NMR (100 MHz, DMSO-d₆): δ =22.6 (4, -CH₃); 30.1 (2, -CH-); 44.8 (2, -CH₂-); 68.3; 68.8; 69.8; 70.4; 79.0 (5, -CH-, -CH₂-); 99.8; 100.0 (2, -OCHO-); 126.5; 128.9; 136.2; 136.6; 141.96; 142.02 (12, aromatic); 174.3 (1, -CON-); HRMS (EI): Calculated for C₂₈H₃₆NO₆ [M-H]⁺ 482.2543, found 482.2534.

### Examples 34-47:

### General procedure for examples 34-46

A 150 mL beaded rim flask, equipped with aluminum heating block, magnetic stirrer and pressure seal was charged with a 3,5:4,6-Dibenzylidene-*L*-gluconic acid methyl ester of one of the Examples 9, 11 and 17 (1.00 Eq.), an amine derivative in methanol (2 N) (15.00 Eq.) or an amine derivative (8.00 Eq.) in methanol (0.75 mol/L). The suspension was stirred for about 16 h at 70 °C and overnight at room temperature until thin layer chromatography (TLC) showed full conversion. The formed powder was isolated by filtration and washed three times with diethyl ether. The product was dried in a vacuum at 110 °C to yield the corresponding substituted 3,5:4,6-Dibenzylidene-*L*-gulonic alkyl amide.

### Example 34: 3,5:4,6-Dibenzylidene-L-gulonic methyl amide

According to the general procedure 3,5:4,6-Dibenzylidene-*L*-gulonic acid methyl ester of Example 9 (0.012 mol, 4.50 g) was reacted with methyl amine in methanol (2 N) (0.175 mol, 87.35 mL). General workup yielded the product as a white powder (4.32 g, 96 %). R_{f} = 0.44 (Ethylacetate - Hexane 4:1); ¹H-NMR (400 MHz, DMSO-d₆): δ = 2.57-2.58 (d, 3, -CH₃); 3.99-4.23 (m, 6, -CH-, -CH₂-); 5.43 (bs, J = x Hz, 1, -OH); 5.66 (s, 1, -OCHO-); 5.69 (s, 1, -OCHO-); 7.37-7.50 (m, 10, aromatic); 7.99-8.00 (bd, 1, -NH-); ¹³C-NMR (100 MHz, DMSO-d₆): δ = 25.9 (1, -CH₃); 68.6; 68.7, 69.7; 70.4; 78.9 (5, -CH-, -CH₂-); 99.6; 99.9 (2, -OCHO-); 125.2; 126.6; 126.7; 128.38; 128.41; 129.1; 138.7; 139.1 (12, aromatic); 172.6 (1, -CON-); HRMS (EI): Calculated for C₂₁H₂₂NO₆ [M-H]⁺ 384.1441, found 384.1441.

### Example 35: 3,5:4,6-Dibenzylidene-L-gulonic ethyl amide

According to the general procedure in two 150 mL beaded rim flasks 3,5:4,6-Dibenzylidene-*L*-gulonic acid methyl ester of Example 9 (0.013 mol, 5.00 g) was reacted with ethyl amine in methanol (2 N) (0.194 mol, 97.05 mL). General workup yielded the product as a white powder (4.90 g, 95 %). R_{f} = 0.49 (Ethylacetate - Hexane 4:1); ¹H-NMR (400 MHz, DMSO-d₆): δ = 0.96 (t, 3, -CH₃); 2.97-3.07 (m, 1, -CH₂-); 3.07-3.17 (m, 1, -CH₂-); 3.98-4.24 (m, 6, -CH-, -CH₂-); 5.34-5.35 (d, J = 5.4 Hz, 1, -OH); 5.66 (s, 1, -OCHO-); 5.68 (s, 1, -OCHO-); 7.35-7.49 (m, 10, aromatic); 8.04 (t, 1, -NH-); ¹³C-NMR (100 MHz, DMSO-d₆): δ =15.1 (1, -CH₃); 33.7 (1, -CH₂-); 68.6; 68.8, 69.8; 70.4; 79.0 (5, -CH-, -CH₂-); 99.5; 99.8 (2, -OCHO-); 126.5; 126.7; 128.3; 128.4; 129.0; 138.7; 139.0 (12, aromatic); 171.8 (1, -CON-); HRMS (EI): Calculated for C₂₂H₂₄NO₆ [M-H]⁺ 398.1604, found 398.1597.

### Example 36: 3,5:4,6-Dibenzylidene-L-gulonic propyl amide

According to the general procedure 3,5:4,6-Dibenzylidene-*L*-gulonic acid methyl ester of Example 9 (0.003 mol, 1.00 g) was reacted with propyl amine (0.021 mol, 1.70 mL) in methanol (15.75 mL). General workup yielded the product as a white powder (0.89 g, 84 %). R_{f} = 0.54 (Ethylacetate - Hexane 4:1); ¹H-NMR (400 MHz, DMSO-d₆): δ = 0.74 (t, 3, -CH₃); 1.30-1.39 (m, 2, -CH₂-); 2.88-2.96 (m, 1, -CH₂-); 3.03-3.12 (m, 1, -CH₂-); 3.98-4.23 (m, 6, -CH-, -CH₂-); 5.33-5.35 (d, J = 5.3 Hz, 1, -OH); 5.65 (s, 1, -OCHO-); 5.68 (s, 1, -OCHO-); 7.35-7.50 (m, 10, aromatic); 8.02 (t, 1, -NH-); ¹³C-NMR (100 MHz, DMSO-d₆): δ = 11.8 (1, -CH₃); 22.7 (2, -CH₂-); 68.7; 68.8; 69.8; 70.4; 79.0 (5, -CH-, -CH₂-); 99.6; 99.9 (2, -OCHO-); 126.6; 126.7; 128.3; 128.4; 129.1; 138.7; 139.1 (12, aromatic); 172.0 (1, -CON-); HRMS (EI): Calculated for C₂₃H₂₆NO₆ [M-H]⁺ 412.1760, found 412.1755.

### Example 37: 3,5:4,6-Dibenzylidene-L-gulonic butyl amide

According to the general procedure 3,5:4,6-Dibenzylidene-L-gulonic acid methyl ester of Example 9 (0.003 mol, 1.00 g) was reacted with butyl amine (0.021 mol, 2.05 mL) in methanol (15.75 mL). General workup yielded the product as a white powder (0.80 g, 83 %). R_{f} = 0.59 (Ethylacetate - Hexane 4:1); ¹H-NMR (400 MHz, DMSO-d₆): δ = 0.74 (t, 3, -CH₃); 1.12-1.21 (m, 2, -CH₂-); 1.27-1.35 (m, 2, -CH₂-); 2.90-2.97 (m, 1, -CH₂-); 3.09-3.18 (m, 1, -CH₂-); 3.98-4.23 (m, 6, -CH-, -CH₂-); 5.32-5.34 (d, J = 5.3 Hz, 1, -OH); 5.65 (s, 1, -OCHO-); 5.68 (s, 1, -OCHO-); 7.34-7.50 (m, 10, aromatic); 8.00 (t, 1, -NH-); ¹³C-NMR (100 MHz, DMSO-d₆): δ = 14.1 (1, -CH₃); 19.9 (1, -CH₂-); 31.7 (1, -CH₂-); 38.4 (1, -CH₂-); 68.7; 68.8; 69.8; 70.4; 79.0 (5, -OCHO-); 99.7; 99.9 (2, -OCHO-); 126.6; 126.7; 128.3; 128.4; 129.1; 138.7; 139.1 (12, aromatic); 172.0 (1, -CON-); HRMS (EI): Calculated for C₂₃H₂₈NO₆ [M-H]⁺ 426.1917, found 426.1912.

### Example 38: 3,5:4,6-Dibenzylidene-L-gulonic octyl amide

According to the general procedure 3,5:4,6-Dibenzylidene-*L*-gulonic acid methyl ester of Example 9 (0.013 mol, 5.00 g) was reacted with octyl amine (0.104 mol, 17.15 mL) in methanol (78.00 mL). General workup yielded the product as a white powder (5.80 g, 93 %). R_{f} = 0.70 (Ethylacetate - Hexane 4:1); ¹H-NMR (400 MHz, DMSO-d₆): δ = 0.84 (t, 3, -CH₃); 1.14-1.25 (m, 10, -CH₂-); 1.31-1.34 (m, 2, -CH₂-); s.89-2.97 (m, 1, -CH₂-); 3.07-3.16 (m, 1, -CH₂-); 3.98-4.23 (m, 6, -CH-, -CH₂-); 5.32-5.33 (d, J = 5.3 Hz, 1, -OH); 5.65 (s, 1, -OCHO-); 5.68 (s, 1, -OCHO-); 7.34-7.50 (m, 10, aromatic); 8.00 (t, 1, -NH-); ¹³C-NMR (100 MHz, DMSO-d₆): δ = 14.4 (1, -CH₃); 22.6; 26.8; 29.0; 29.2; 29.6; 31.8; 38.8 (7, -CH₂-); 68.7; 68.8; 69.8; 70.4; 79.0 (5, -CH-, -CH₂-); 99.6; 99.9 (2, -OCHO-); 126.6; 126.7; 128.3; 128.4; 129.0; 129.1; 138.7; 139.0 (10, aromatic); 171.9 (1, -CON-); HRMS (EI): Calculated for C₂₈H₃₆NO₆ [M-H]⁺ 482.2543, found 482.2534.

### Example 39: 3,5:4,6-Dibenzylidene-L-gulonic dodecyl amide

According to the general procedure 3,5:4,6-Dibenzylidene-*L*-gulonic acid methyl ester of Example 9 (0.004 mol, 1.50 g) was reacted with dodecyl amine (0.031 mol, 7.20 mL) in methanol (23.25 mL). General workup yielded the product as a white powder (1.58 g, 75 %). R_{f} = 0.75 (Ethylacetate - Hexane 4:1); ¹H-NMR (400 MHz, DMSO-d₆): δ = 0.85 (t, 3, -CH₃); 1.13-1.32 (m, 20, -CH₂-); 2.89-2.97 (m, 1, -CH₂-); 3.08-3.16 (m, 1, -CH₂-); 3.98-4.23 (m, 6, -CH-, -CH₂-); 5.32-5.33 (d, J = 5.3 Hz, 1, -OH); 5.65 (s, 1, -OCHO-); 5.68 (s, 1, -OCHO-); 7.34-7.49 (m, 10, aromatic); 8.00 (t, 1, -NH-); ¹³C-NMR (100 MHz, DMSO-d₆): δ =14.4 (1, -CH₃); 22.6; 26.8; 29.2; 29.3; 29.4; 29.5; 29.5; 29.6; 31.8; 38.8 (11, -CH₂-); 68.7; 68.8; 69.8; 70.4; 79.0 (5, -CH-, -CH₂-); HRMS (EI): Calculated for C₃₂H₄₄NO₆ [M-H]⁺ 538.3169, found 538.3167.

### Example 40: 3,5:4,6-Bis-(3,4-Dimethylbenzylidene)-L-gulonic methyl amide

According to the general procedure 3,5:4,6-Bis-(3,4-Dimethylbenzylidene)-*L*-gulonic acid methyl ester of Example 11 (0.009 mol, 3.80 g) was reacted with methyl amine in methanol (2 N) (0.123 mol, 64.41 mL). General workup yielded the product as a white powder (3.57 g, 94 %). R_{f} = 0.00 (Ethylacetate - Hexane 4:1);
¹H-NMR (400 MHz, DMSO-d₆): δ = 2.21-2.23 (bd, 12, -CH₃); 2.56-2.57 (d, 3, -CH₃); 3.92-4.18 (m, 6, -CH-, -CH₂-); 5.35-5.37 (d, J = 5.3 Hz, 1, -OH); 5.55 (s, 1, -OCHO-); 5.59 (s, 1, -OCHO-); 7.12-7.23 (m, 6, aromatic); 7.98-7.99 (bd, 1, -NH-); HRMS (EI): Calculated for C₂₅H₃₀NO₆ (M-H)⁺ 440.2073, found 440.2066.

### Example 41: 3,5:4,6-Bis-(3,4-Dimethylbenzylidene)-L-gulonic ethyl amide

According to the general procedure 3,5:4,6-Bis-(3,4-Dimethylbenzylidene)-*L*-gulonic acid methyl ester of Example 11 (0.009 mol, 3.80 g) was reacted with ethyl amine in methanol (2 N) (0.123 mol, 64.41 mL). General workup yielded the product as a white powder (3.72 g, 95 %). R_{f} = 0.00 (Ethylacetate - Hexane 4:1);
¹H-NMR (400 MHz, DMSO-d₆): δ =0.96-1.00 (t, 3, -CH₃); 2.21-2.23 (m, 12, -CH₃); 2.98-3.17 (m, 2, -CH₂-); 3.92-4.16 (m, 6, -CH-, -CH₂-); 5.30 (bd, J = 5.3 Hz, 1, -OH); 5.57 (s, 1, -OCHO-); 5.59 (s, 1, -OCHO-); 7.11-7.22 (m, 6, aromatic); 8.05 (t, 1, -NH-); HRMS (EI): Calculated for C₂₆H₃₂NO₆ (M-H)⁺ 454.2230, found 454.2225.

### Example 42: 3,5:4,6-Di-(4-Propylbenzylidene)-L-gulonic methyl amide

According to the general procedure 3,5:4,6-Di-(4-Propylbenzylidene)-L-gulonic acid ethyl ester of Example 17 (0.008 mol, 4.00 g) was reacted with ethyl amine in methanol (2 N) (0.124 mol, 61.91 mL). General workup yielded the product as a white powder (3.61 g, 93 %). R_{f} = 0.61 (Ethylacetate - Hexane 4:1);
¹H-NMR (400 MHz, DMSO-d6): δ = 0.85-0.90 (t, 6, -CH₃); 1.53-1.62 (m, 4, -CH₂-); 2.53-2.56 (m, 7, -CH₂-, -CH₃); 3.94-4.20 (m, 6, -CH-, -CH₂-); 5.37-5.39 (d, J = 5.3 Hz, 1, -OH); 5.60 (s, 1, -OCHO-); 5.63 (s, 1, -OCHO-); 7.17-7.20 (m, 4, aromatic); 7.31-7.33 (d, 2, aromatic); 7.37-7.39 (d, s, aromatic); 7.96-7.97 (bd, 1, -NH-); HRMS (EI): Calculated for C₂₇H₃₄NO₆ (M-H)⁺ 468.2386, found 468.2378.

### Example 43: 3,5:4,6-Di-(4-Propylbenzylidene)-L-gulonic ethyl amide

According to the general procedure 3,5:4,6-Di-(4-Propylbenzylidene)-*L*-gulonic acid ethyl ester of Example 17 (0.008 mol, 4.00 g) was reacted with ethyl amine in methanol (2 N) (0.124 mol, 61.91 mL). General workup yielded the product as a white powder (3.67 g, 92 %). R_{f} = 0.59 (Ethylacetate - Hexane 4:1); ¹H-NMR (400 MHz, DMSO-d₆): δ = 0.85-0.89 (m, 6, -CH₃); 0.96 (t, 3, -CH₃); 1.52-1.62 (m, 4, -CH₂-); 2.52-2.57 (m, 4, -CH₂-); 2.95-3.04 (m, 1, -CH₂-); 3.08-3.14 (m, 1, -CH₂-); 5.30-5.32 (d, 1, -OH); 5.61 (s, 1, -OCHO-); 5.63 (s, 1, -OCHO-); 7.18 (t, 4, aromatic); 7.31-7.33 (d, 2, aromatic); 7.37-7.39 (d, 2, aromatic); 8.02 (t, 1, -NH-); HRMS (EI): Calculated for C₂₈H₃₆NO₆ [M-H]⁺ 482.2543, found 482.2533.

### Example 44: 3,5:4,6-Dibenzylidene-L-gulonic hydroxyl amide

According to the general procedure relevant 3,5:4,6-Dibenzylidene-*L*-gulonic acid methyl ester of Example 9 (0.003 mol, 1.00 g) was reacted with hydroxylamine in water (50 %) (0.02 mol, 1.06 mL) and 40.00 mL methanol. General workup yielded the product as a white powder (0.59 g, 59 %). R_{f} = 0.00-0.20 (Ethylacetate - Hexane 4:1); ¹H-NMR (400 MHz, DMSO-d₆): δ = 3.98-4.22 (m, 6, -CH-, -CH₂-); 5.53-5.55 (d, 1, -OH); 5.64-5.67 (d, 2, -OCHO-); 7.36-7.51 (m, 10, aromatic); 8.84 (s, 1, -NH-), 10.67 (s, 1, -OH).

### Example 45: 3,5:4,6-Dibenzylidene-L-gulonic monoethanol amide

According to the general procedure 3,5:4,6-Dibenzylidene-*L*-gulonic acid methyl ester (Example 9, 0.003 mol, 1.00 g) was reacted with monoethanolamine (0.020 mol, 1.17 mL) in methanol (40.00 mL). General workup yielded the product as a white powder (0.71 g, 66 %). R_{f} = 0.00-0.15 (Ethylacetate - Hexane 4:1); ¹H-NMR (400 MHz, DMSO-d₆): δ = 3.09-3.17 (m, 2, -CH₂-); 3.32-3.37 (m, 2, -CH₂-); 3.98-4.18 (m,6, -CH-, -CH₂-); 4.59 (t, 1, -OH); 5.39-5.40 (d, 1, -OH); 5.66-5.68 (d, 2, -OCHO-); 7.34-7.49 (m, 10, aromatic); 8.00 (t, 1, -NH).

### Example 46: 3,5:4,6-Dibenzylidene-L-gulonic hydrazide

According to the general procedure 3,5:4,6-Dibenzylidene-*L*-gulonic acid methyl ester of Example 9 (0.002 mol, 0.70 g) was reacted with hydrazine (0.027 mol, 2.65 mL) in methanol (40.00 mL). General workup yielded the product as a white powder (0.59 g, 84 %). R_{f} = 0.00 (Ethylacetate - Hexane 4:1); ¹H-NMR (400 MHz, DMSO-d₆): δ = 3.98-4.23 (m, 8, -CH-, -CH₂-, -NH₂); 5.46-5.48 (d, 1, -OH); 5.64 (s, 1, -OCHO-); 5.67 (s, 1, -OCHO-); 7.36-7.50 (m, 10, aromatic); 9.25 (bs, 1, -NH).

### Example 47: 3,5:4,6-Dibenzylidene-L-gulonic acid sodium salt

3,5:4,6-Dibenzylidene-*L*-gulonic acid methyl ester of Example 9 (0.010 mol, 4.00 g) was treated with NaOH (0.010 mol, 0.41 g) in 34.00 mL water. After stirring for 3 h at 70 °C the mixture was cooled down and frozen with liquid nitrogen. Lyophilization yielded the product as a white powder (4.08 g, 100 %). R_{f} = 0.00 -0.10 (Ethylacetate - Hexane 4:1); ¹H-NMR (400 MHz, DMSO-d₆): δ = 3,62-4.23 (m, 7, -CH, -CH₂, -OH); 5.61 (s, 2, -OCHO-); 7.33-7.50 (m, 10, aromatic); HRMS (EI): Calculated for C₂₆H₃₅O₇Si₂ [M-H]⁺ 515.1921, found 515.1918.

### Application:

### Example 48: Application of the compounds of examples 9-47:

### Compounding and injection molding:

Compositions containing various levels of an acetal of Examples 9-47, 0.10 wt.-% Irganox 1010, 0.10 wt.-% Irgafos 168 and 0.10 wt.-% calcium stearate and the polypropylene homopolymer Moplen HP 500 N (MFR = 12 g/10 min, Mₙ = 47 kg/mol, M_{w} = 393 kg/mol) or polypropylene random copolymer Moplen RP 310 M (MFR 8.5 g/10 min) were compounded at 100 rpm in three cycles in a single screw extruder (Weber ET20, 20 mm, 25D) at 220°C. Afterwards the mixtures were discharged and pelletized. Control samples were treated accordingly.

Injection molding was performed with an Arburg Allrounder 2200 S 250-60 (22 mm, 20D). The previously pelletized, compounded granulate was melted at 220°C and injection molded in a polished droplet formed mold. The mold temperature was 50°C. The collected test specimen had a thickness of 1.0 mm.

### Characterization of polymer compositions:

Differential scanning calorimetry (*DSC*) of the polymer compositions obtained in accordance with Example 48 was performed on a Mettler Toledo DSC 3+ under nitrogen atmosphere in an alumina 40 µl crucible with a standard heating or cooling rate of 10 K/min. Prior to recording of thermograms, the samples were held at 230°C for 5 min to erase "thermal history" and prevent self-seeding of the used polymer. Each analysis refers to a two-cycle heating-cooling experiment. The sample weight was 10 mg. Reported crystallization temperatures (T_{c,p}) refer to the peak temperatures of 2^{nd} heating and 1^{st} crystallization run in the corresponding thermograms.

The standard optical characteristics "haze" and "clarity" were measured for the injection molded discs with a "Haze-Gard Plus" instrument (BYK Gardner GmbH), which conforms to ASTM D-1003. Total transmittance is the ratio of total transmitted light to incident light. It is reduced by reflectance and absorbance. Haze is defined as that portion of visible light that is scattered at wider angles (2.5° < θ < 90°) and is a measure for the turbidity of a sample. Clarity, usually refers to the scattering contribution at small angles (θ < 2.5°) and is related to the sharpness of an object when viewed through the sample. The reported values are the average of at least three measured samples. Typically, the standard deviations for the measured three samples were below 0.5 % for haze and clarity.

The crystallization temperature (T_{c,p}), haze and clarity of the polymer compositions obtained according to Example 48 are summarized in Table 1, 2, 3 and 4 (polymer Moplen HP 500 N) and in Table 5 (copolymer Moplen RP 310 M).

**Table 1: Polymer: Moplen HP 500 N**

| Example | Con. [wt%] | T_{c,p} [°C] | Haze [%] | Clarity [%] |
|---|---|---|---|---|
| Control | 0.00 | 113.4 | 63.9 | 44.1 |
| Ex 9 (comparative) | 0.15 | 114.1 | 59.4 | 45.3 |
| | 0.30 | 114.4 | 59.5 | 45.1 |
| | 0.60 | 126.6 | 63.3 | 43.0 |
| Ex 10 | 0.30 | 123.3 | 69.0 | 63.0 |
| | 0.45 | 125.8 | 59.8 | 81.9 |
| | 0.60 | 124.1 | 45.2 | 88.2 |
| Ex 11 | 0.30 | 128.6 | 67.3 | 54.1 |
| | 0.45 | 127.7 | 61.2 | 73.9 |
| | 0.60 | 128.6 | 45.6 | 88.7 |
| | 0.75 | 128.6 | 38.1 | 92.5 |
| Ex 12 | 0.30 | 131.0 | 72.5 | 48.0 |
| | 0.45 | 130.8 | 55.9 | 70.6 |
| | 0.60 | 125.9 | 41.7 | 82.9 |
| Ex 13 | 0.15 | 113.6 | 60.6 | 42.0 |
| | 0.30 | 119.6 | 64.0 | 43.6 |
| | 0.60 | 122.4 | 50.6 | 65.2 |
| Ex 14 | 0.30 | 115.1 | 65.0 | 52.2 |
| Ex 15 | 0.30 | 115.0 | 67.9 | 49.6 |
| | 0.60 | 115.5 | 72.5 | 82.1 |
| Ex 16 | 0.30 | 114.7 | 62.2 | 42.1 |
| | 0.45 | 115.1 | 63.0 | 41.8 |
| Ex 17 | 0.30 | 114.4 | 62.6 | 46.8 |

**Table 2: Polymer: Moplen HP 500 N**

| Example | Con. [wt%] | T_{c,p} [°C] | Haze [%] | Clarity [%] |
|---|---|---|---|---|
| Ex 18 (comparative) | 0.30 | 119.4 | 72.0 | 80.5 |
| | 0.60 | 124.4 | 83.8 | 68.0 |
| Ex 19 | 0.30 | 120.5 | 59.5 | 92.0 |
| Ex 20 | 0.30 | 121.7 | 56.1 | 92.7 |
| Ex 21 | 0.30 | 121.9 | 56.0 | 92.6 |
| | 0.45 | 125.3 | 46.0 | 93.7 |
| | 0.60 | 125.0 | 45.5 | 93.8 |
| Ex 22 | 0.03 | 114.9 | 64.1 | 45.8 |
| | 0.15 | 122.6 | 51.6 | 93.3 |
| | 0.30 | 124.1 | 44.8 | 94.2 |
| | 0.45 | 123.6 | 51.2 | 93.5 |
| Ex. 23 | 0.30 | 117.1 | 70.1 | 72.4 |
| | 0.60 | 118.2 | 63.2 | 88.5 |
| Ex. 24 | 0.30 | 117.3 | 69.2 | 71.5 |
| | 0.60 | 118.2 | 72.1 | 80.9 |
| Ex. 25 | 0.30 | 117.4 | 67.7 | 78.7 |
| | 0.60 | 118.0 | 56.6 | 92.0 |

**Table 3: Polymer: Moplen HP 500 N**

| Example | Con. [wt%] | T_{c,p} [°C] | Haze [%] | Clarity [%] |
|---|---|---|---|---|
| Ex 26 (comparative) | 0.03 | 115.2 | 61.3 | 47.7 |
| | 0.15 | 119.5 | 65.4 | 50.8 |
| | 0.30 | 126.3 | 70.2 | 55.8 |
| | 0.45 | 120.4 | 63.3 | 69.3 |
| | 0.60 | 123.2 | 49.3 | 90.4 |
| Ex 27 | 0.15 | 121.8 | 38.9 | 93.8 |
| | 0.30 | 123.1 | 26.6 | 93.3 |
| | 0.45 | 123.1 | 35.5 | 88.7 |
| | 0.60 | 122.4 | 48.5 | 81.8 |
| Ex 28 | 0.15 | 124.3 | 34.1 | 94.2 |
| | 0.225 | 124.5 | 24.8 | 94.6 |
| | 0.30 | 124.0 | 22.8 | 94.4 |
| | 0.375 | 123.4 | 22.8 | 93.9 |
| | 0.45 | 123.3 | 22.7 | 93.6 |
| | 0.60 | 122.6 | 25.7 | 92.7 |
| Ex 29 | 0.15 | 122.0 | 40.9 | 93.1 |
| | 0.30 | 122.5 | 27.9 | 94.0 |
| Ex 30 | 0.03 | 112.8 | 60.4 | 41.3 |
| | 0.30 | 122.2 | 46.1 | 93.6 |
| | 0.45 | 122.2 | 40.3 | 92.1 |
| | 0.60 | 128.6 | 39.9 | 90.7 |
| Ex 31 | 0.30 | 118.7 | 62.7 | 87.4 |
| | 0.45 | 122.4 | 63.5 | 83.1 |
| | 0.60 | 116.9 | 41.7 | 92.6 |
| Ex 32 | 0.15 | 117.5 | 59.2 | 91.6 |
| | 0.30 | 122.3 | 30.6 | 94.3 |
| | 0.45 | 121.4 | 26.2 | 94.8 |
| | 0.60 | 122.0 | 26.2 | 94.5 |
| Ex 33 | 0.30 | 115.7 | 61.0 | 90.0 |
| | 0.45 | 123.1 | 37.4 | 94.2 |
| | 0.60 | 126.8 | 29.9 | 94.6 |

**Table 4: Polymer: Moplen HP 500 N**

| Example | Con. [wt%] | T_{c,p} [°C] | Haze [%] | Clarity [%] |
|---|---|---|---|---|
| Ex 34 (comparative) | 0.15 | 117.1 | 65.6 | 66.8 |
| | 0.30 | 128.3 | 61.5 | 79.5 |
| | 0.45 | 127.7 | 53.7 | 78.8 |
| | 0.60 | 127.8 | 41.7 | 77.3 |
| Ex 35 (comparative) | 0.30 | 116.2 | 67.6 | 67.6 |
| | 0.45 | 123.2 | 49.3 | 71.1 |
| | 0.60 | 123.1 | 42.1 | 83.1 |
| Ex 36 (comparative) | 0.15 | 116.1 | 65.2 | 52.3 |
| | 0.30 | 121.7 | 68.6 | 58.6 |
| | 0.45 | 124.8 | 69.0 | 70.0 |
| | 0.60 | 124.2 | 55.9 | 71.5 |
| Ex 37 (comparative) | 0.03 | 115.0 | 62.9 | 45.0 |
| | 0.30 | 116.3 | 68.0 | 62.0 |
| | 0.60 | 125.7 | 52.5 | 77.1 |
| Ex 38 (comparative) | 0.30 | 116.1 | 67.3 | 59.9 |
| | 0.45 | 116.2 | 64.7 | 53.6 |
| | 0.60 | 115.1 | 62.4 | 59.3 |
| Ex 39 (comparative) | 0.03 | 114.2 | 63.1 | 47.7 |
| | 0.30 | 116.8 | 67.2 | 63.1 |
| Ex 40 | 0.03 | 115.1 | 66.5 | 50.4 |
| | 0.15 | 124.1 | 61.7 | 93.4 |
| | 0.30 | 124.7 | 86.5 | 67.6 |
| Ex 41 | 0.30 | 125.7 | 60.3 | 91.3 |
| | 0.45 | 126.9 | 38.9 | 94.3 |
| | 0.60 | 126.5 | 29.8 | 94.2 |
| Ex 42 | 0.15 | 126.0 | 65.9 | 86.6 |
| | 0.30 | 122.5 | 45.1 | 90.2 |
| | 0.45 | 124.2 | 43.1 | 85.4 |
| Ex 43 | 0.30 | 121.1 | 64.1 | 78.9 |
| | 0.45 | 130.3 | 57.4 | 91.9 |
| | 0.60 | 130.9 | 36.0 | 93.5 |
| Ex 44 (comparative) | 0.03 | 115.0 | 63.9 | 52.3 |
| | 0.15 | 116.2 | 68.5 | 61.8 |
| | 0.30 | 116.1 | 70.8 | 62.9 |
| Ex 45 (comparative) | 0.03 | 115.2 | 61.4 | 44.2 |
| | 0.15 | 118.5 | 68.5 | 52.3 |
| | 0.30 | 118.9 | 71.9 | 52.3 |
| Ex 46 (comparative) | 0.03 | 115.4 | 61.1 | 54.5 |
| | 0.30 | 118.0 | 63.5 | 65.1 |
| | 0.60 | 121.8 | 45.0 | 93.9 |
| Ex 47 (comparative) | 0.03 | 119.3 | 71.9 | 28.3 |
| | 0.15 | 120.6 | 83.1 | 29.8 |
| | 0.30 | 120.4 | 86.1 | 39.9 |
| | 0.45 | 121.2 | 88.4 | 41.9 |
| | 0.60 | 122.3 | 90.4 | 43.6 |

**Table 5: Polymer: Moplen RP 310 M**

| Example | Con. [wt%] | T_{c,p} [°C] | Haze [%] | Clarity [%] |
|---|---|---|---|---|
| Control | 0.00 | 109.6 | 55.10 | 71.30 |
| Ex 11 | 0.03 | 109.9 | 56.40 | 70.70 |
| | 0.15 | 121.0 | 55.40 | 96.10 |
| | 0.30 | 120.2 | 55.80 | 84.30 |
| | 0.45 | 120.6 | 39.40 | 91.90 |
| | 0.60 | 120.6 | 32.20 | 94.10 |
| | 0.75 | 120.8 | 28.00 | 94.90 |
| Ex 28 | 0.03 | 108.7 | 54.30 | 77.60 |
| | 0.15 | 120.3 | 20.60 | 95.30 |
| | 0.30 | 120.0 | 16.80 | 95.00 |
| | 0.45 | 119.2 | 19.50 | 94.20 |
| | 0.60 | 117.1 | 20.10 | 94.00 |

## Claims

1. A nucleating agent of formula (I) 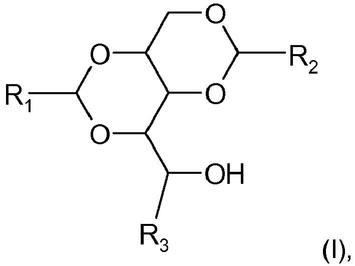 wherein
residues R₁ and R₂ are independently selected from substituted phenyl groups, wherein the substituents of said substituted phenyl groups are independently from each other selected from the group consisting of (C₁-C₄)-alkyl-;
and wherein
R₃ is a residue of formula (II), (III) or (IV), 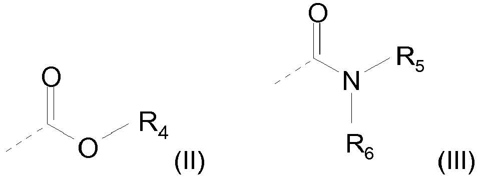 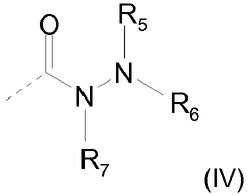 wherein
R₄, R₅, R₆, and R₇ are independently from each other hydrogen or (C₁-C₄)-alkyl-.

2. The nucleating agent of claim 1, **characterized in, that** the residues R₁ and R₂ are independently selected from monosubstituted or disubstituted phenyl groups, where the substituents of said monosubstituted or disubstituted phenyl groups are independently from each other selected from the group consisting of (C₁-C₄)-alkyl-.

3. The nucleating agent of claim 1 or 2, **characterized in, that** the residues R₁ and R₂ are independently selected from group consisting 4-methylphenyl, 4-ethylphenyl, 4-propylphenyl, 4-butylphenyl, 3-methylphenyl, 2,4-dimethylphenyl, 2,5-dimethylphenyl, 3,4-dimethylphenyl-, and 3,5-dimethylphenyl.

4. The nucleating agent of any one of claims 1 to 3, **characterized in, that** the nucleating agent is a L-gulonic acid derivative of formula (Ia)

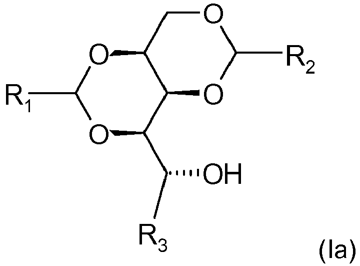

5. The nucleating agent of any one of claims 1 to 4, **characterized in, that** R₄, R₅, and R₇ are hydrogen; and R₆ is hydrogen, methyl or ethyl.

6. The nucleating agent of any one of claims 1 to 5, **characterized in, that** in the case the compound of formula (I) or (Ia) has a residue R₃ of formula (II), 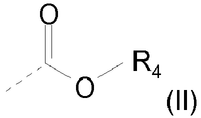 R₄ = H; the residue R₁ = R₂ and the residues R₁ and R₂ are selected from the group consisting of 4-methylphenyl, 3,4-dimethylphenyl, 3,5-dimethylphenyl, and 2,4-dimethylphenyl; or
in the case the compound of formula (I) or (Ia) has a residue R₃ of formula (III) 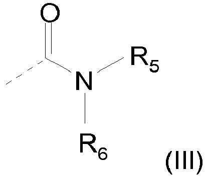 R₅ = H and R₆ = H, methyl or ethyl; the residue R₁ = R₂ and the residues R₁ and R₂ are selected from the group consisting of 4-methylphenyl, 4-ethylphenyl, 4-propylphenyl, 4-butylphenyl, 3,4-dimethylphenyl, 3,5-dimethylphenyl, and 2,4-dimethylphenyl; or in the case the compound of formula (Ia) has a residue R₃ of formula (IV) 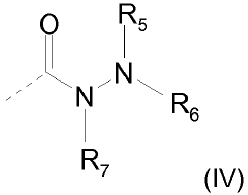 R₅ = H, R₆ = H, methyl or ethyl, and R₇ = H; the residue R₁ = R₂ and the residues R₁ and R₂ are selected from the group consisting of 4-methylphenyl, 4-ethylphenyl, 4-propylphenyl, 4-butylphenyl, 3,4-dimethylphenyl, 3,5-dimethylphenyl, and 2,4-dimethylphenyl.

7. The nucleating agent of claim 1, **characterized in, that** the nucleating agent is selected from the group consisting of 3,5:4,6-Di-(4-Methylbenzylidene)-L-gulonic acid methyl ester, 3,5:4,6-Bis-(3,4-Dimethylbenzylidene)-L-gulonic acid methyl ester, 3,5:4,6-Bis-(3,5-Dimethylbenzylidene)-L-gulonic acid methyl ester, 3,5:4,6-Di-(4-Propylbenzylidene)-L-gulonic acid methyl ester, 3,5:4,6-Di-(4-Methylbenzylidene)-L-gulonic acid, 3,5:4,6-Bis-(3,4-Dimethylbenzylidene)-L-gulonic acid, 3,5:4,6-Bis-(3,5-Dimethylbenzylidene)-L-gulonic acid, 3,5:4,6-Bis-(2,4-Dimethylbenzylidene)-L-gulonic acid, 3,5:4,6-Bis-(2,5-Dimethylbenzylidene)-L-gulonic acid, 3,5:4,6-Di-(4-Propylbenzylidene)-L-gulonic acid, 3,5:4,6-Di-(4-Isobutylbenzylidene)-L-gulonic acid, 3,5:4,6-Di-(4-Methylbenzylidene)-L-gulonic amide, 3,5:4,6-Bis-(3,4-Dimethylbenzylidene)-L-gulonic amide, 3,5:4,6-Bis-(3,5-Dimethylbenzylidene)-L-gulonic amide, 3,5:4,6-Bis-(2,4-Dimethylbenzylidene)-L-gulonic amide, 3,5:4,6-Bis-(2,5-Dimethylbenzylidene)-L-gulonic amide, 3,5:4,6-Di-(4-Propylbenzylidene)-L-gulonic amide, 3,5:4,6-Di-(4-Isobutylbenzylidene)-L-gulonic amide, 3,5:4,6-Bis-(3,4-Dimethylbenzylidene)-L-gulonic methyl amide, 3,5:4,6-Bis-(3,4-Dimethylbenzylidene)-L-gulonic ethyl amide, 3,5:4,6-Di-(4-Propylbenzylidene)-L-gulonic methyl amide, and 3,5:4,6-Di-(4-Propylbenzylidene)-L-gulonic ethyl amide.

8. Use of one or more nucleating agents of any one of claims 1 to 7 as clarifier for polymer compositions.

9. The use of one or more nucleating agents according to claim 8 **characterized in, that** the polymer composition comprises at least one polymer selected from the group consisting of olefinic homopolymers, olefinic copolymers, polyamides, polystyrene, polyesters, polyketones, polyoxyalkylenes, poly(phenylene)sulfide, polyvinylchloride.

10. The use of one or more nucleating agents according to claim 8 or 9, **characterized in, that** the polymer composition comprises a polymer selected from the group consisting of olefinic homopolymers derived from aliphatic olefins with 2 to 6 carbon atoms or olefinic copolymers derived from at least one aliphatic olefin with 2 to 6 carbon atoms and one or more ethylenically unsaturated comonomers, polyamide-6,6, and polyamide-6 or mixtures thereof.

11. The use of one or more nucleating agents according to any one of claims 8 to 10, **characterized in, that** the polymer composition comprises a polymer selected from polyethylene, polytetrafluoroethylene, polypropylene, polybutylene, polyisobutylene, polyisoprene, polybutadiene, polystyrene, polyethylene terephthalate, polypropylene terephthalate, polybutylene terephthalate, copolymers of ethylene and an ethylenically unsaturated comonomer selected from the group of propylene, 1-butylene, isobutylene, 1-pentylene, 1-hexylene, isoprene, (C₃-C₇)-cycloalkene, norbornene and styrene, copolymers of propylene and an ethylenically unsaturated comonomer selected from the group of ethylene, 1-butylene, isobutylene, 1-pentylene, 1-hexylene, isoprene, (C₃-C₇)-cycloalkene, norbornene and styrene; or mixtures thereof.
